# FASCICULE DE BREVET EUROPEEN

(11) **EP 2 643 301 B1**
(45) Date de publication et mention de la délivrance du brevet: **04.10.2017**
(21) Numéro de dépôt: 11788439.5
(22) Date de dépôt: 24.11.2011
(51) Int. Cl.: C07D 213/82, C07F 9/02, A61K 31/44, A61P 35/00

(54) **COMPLEXE DE TECHNETIUM 99M EN TANT QU'OUTIL DE DIAGNOSTIC IN VIVO DES TUMEURS CANCEREUSES**
TECHNETIUM-99M-KOMPLEX ALS WERKZEUG ZUR IN-VIVO-DIAGNOSE VON KARZINÖSEN TUMOREN
TECHNETIUM-99M COMPLEX AS A TOOL FOR THE IN VIVO DIAGNOSIS OF CANCEROUS TUMOURS

(30) Priorité: 24.11.2010 FR 1059690
(43) Date de publication de la demande: 02.10.2013
(73) Titulaire: Pierre Fabre Médicament, 92100 Boulogne-Billancourt (FR); Centre National de la Recherche Scientifique (C.N.R.S.), 75016 Paris (FR)
(72) Inventeur: GUMINSKI, Yves, F-81090 Lagarrigue (FR); IMBERT, Thierry, F-81290 Viviers les Montagnes (FR); PESNEL, Sabrina, F-45160 Olivet (FR); GUILBAUD, Nicolas, F-31400 Toulouse (FR); LE PAPE, Alain, F-37130 Lignieres de Touraine (FR); LERONDEL, Stéphanie, F-45000 Orléans (FR)
(74) Mandataire: Regimbeau
(86) Numéro de dépôt international: PCT/EP2011/070981
(87) Numéro de publication internationale: WO 2012/069608

(56) Documents cités:
- WO-A2-98/31399
- BRIDGER G J ET AL: "A COMPARISON OF CLEAVABLE AND NONCLEAVABLE HYDRAZINOPYRIDINE LINKERS FOR THE 99MTC LABELING OF FAB' MONOCLONAL ANTIBODY FRAGMENTS", BIOCONJUGATE CHEMISTRY, ACS, WASHINGTON, DC, US, vol. 7, no. 2, 1 mars 1996 (1996-03-01), pages 255-264, XP000558423, ISSN: 1043-1802, DOI: DOI:10.1021/BC960008R

## Description

La présente invention concerne des dérivés radio-pharmaceutiques constitués de polyamines conjuguées avec l'HYNIC (*hydrazinonicotinamide*) pouvant complexer le Technétium 99m, leur procédé de préparation et leur utilisation sous forme d'agents d'imagerie permettant de mettre en évidence le système de transport des polyamines dans les cellules cancéreuses pour permettre la sélection des patients porteurs de telles tumeurs en vue d'adapter leur traitement.

Les possibilités devenues incontournables offertes par les applications des traceurs et de la radioactivité en milieu biologique et en médecine ont été l'un des facteurs essentiels du progrès médical au cours du XXe siècle. Les techniques utilisant la radioactivité élargissent les possibilités de diagnostic pour détecter et mieux guérir les maladies : c'est la médecine nucléaire. Au lieu de faire passer les rayonnements à travers l'ensemble du corps comme dans la radiographie, on introduit dans l'organisme une petite quantité de molécules marquées par un radio-isotope émetteur de rayonnement gamma. Ce traceur reconnaîtra certaines cibles d'intérêt qui seront détectées ensuite par une γ-caméra.

Le cancer est encore une des principales causes de mortalité du monde occidental. Les moyens de lutte que sont la prévention, la chirurgie, la radiothérapie, l'immunothérapie et la chimiothérapie ne permettent pas encore, dans de nombreux cas, d'éradiquer la maladie. Les raisons de cet échec reposent en partie sur la difficulté d'identifier la cellule tumorale et de la traiter sélectivement sans trop endommager les tissus sains.

L'imagerie par scintigraphie *in vivo* est un outil permettant d'identifier les tissus tumoraux par rapport aux tissus sains. Cette approche de marquage par radioactivité est capable de détecter des tumeurs de taille extrêmement petite. Elle est basée sur l'obtention d'une cartographie *in vivo* issue de la détection externe du rayonnement gamma émis par les radio-isotopes au cours de leur désintégration au sein des tissus en donnant une image en 3D.

Le radio-isotope idéal pour l'imagerie TEMP (Tomographie d'Emission MonoPhotonique) est le ^{99m}Tc, en raison de son faible coût par dose et de sa disponibilité au moyen de systèmes générateurs disponibles dans les milieux hospitaliers. Plus de 80 % de toutes les études d'imagerie en médecine nucléaire de diagnostic dans le monde sont effectuées en utilisant ce radio-isotope (^{99m}Tc). Cet élément radioactif émet un rayonnement y particulièrement bien adapté à l'imagerie médicale et à son utilisation extensive pour le diagnostic médical. Le rayonnement y émis par l'isotope est détecté *in vivo* à l'aide d'une gamma-caméra pour former des images scintigraphiques.

Le Technétium 99m est particulièrement intéressant pour les applications médicales : la radiation émise par désintégration de cet isotope a la même longueur d'onde que les rayons X utilisés en radiographie classique, ce qui lui confère une longueur de pénétration adaptée tout en causant des dégâts minimes pour un photon gamma. De plus, la demi-vie très courte de cet isotope conjuguée à la demi-vie relativement longue de l'isotope fils Tc-99 lui permet d'être éliminé du corps avant de se désintégrer à nouveau. Sa demi-vie est de 6 heures, ce qui donne suffisamment de temps pour l'acquisition des images et permet une élimination rapide de la radioactivité. Ceci permet de réaliser un diagnostic nucléaire au prix de l'introduction d'une dose relativement faible de radiation dans l'organisme.

De plus, il est facilement disponible dans les hôpitaux grâce à un générateur de technétium. Le générateur contient du molybdène 99 radioactif, attaché (absorbé) sur une colonne d'alumine. Le molybdène se désintègre pour donner du ^{99m}Tc, qui est récupéré par rinçage de la colonne dans une solution physiologique sous la forme de pertechnétate de sodium (Na⁺TcO4⁻).

Le Technétium 99m peut être utilisé sous une forme moléculaire simple (^{99m}Tc-Pertechnétate) mais est plus fréquemment associé à des molécules leur conférant des propriétés particulières. Par exemple, le Technétium 99m complexé à de l'acide diéthylènetriaminepentacétique (^{99m}Tc-DTPA) éliminé exclusivement par filtration glomérulaire est un bon marqueur de la fonction rénale. Le Technétium 99m lié au méthyldiphosphonate (^{99m}Tc-MDP) a une affinité particulière pour le tissu osseux. Ce composé radio-pharmaceutique est utilisé pour déceler des zones d'activité ostéoblastique. L'intensité du rayonnement permet alors de déterminer la concentration en composé radio-pharmaceutique dans le liquide biologique ou l'organe étudié.

Parmi les composés complexés au Technétium 99m, on peut citer également l'H.M.P.A.O. (hexa-méthyl-propylène-amine-oxime) qui sert à étudier les accidents vasculaires cérébraux, l'épilepsie partielle, certains états démentiels et la souffrance cérébrale de l'enfant ; le sesta-M.I.B.I. (méthoxy-iso-butyl-isonitrile) utilisé pour le diagnostic de l'ischémie myocardique (arrêt ou insuffisance de l'apport de sang et d'oxygène au coeur). Le Technétium 99m peut également se complexer avec les globules rouges permettant d'étudier la contraction des ventricules cardiaques, ainsi qu'avec les microparticules d'albumine, qui servent à étudier la vascularisation des poumons, en particulier dans le diagnostic des embolies. On peut citer également le DMSA (acide di-mercaptosuccinique) ; le DTPA (acide diéthylène-triamino-pentacétique) ou le M.A.G.3 (mercapto-acétyl-tri-glycine) utilisés pour l'évaluation fonctionnelle séparée de chaque rein dans de nombreuses pathologies rénales, le bilan préchirurgical de l'ablation d'un rein et l'exploration des malformations rénales congénitales ; l'IDA (acide imino-diacétique) qui sert à visualiser les voies biliaires en chirurgie digestive ; les colloïdes marqués pour l'étude des voies et ganglions lymphatiques et le repérage du ganglion sentinelle (premier ganglion touché par l'extension tumorale). Enfin, des aliments marqués au Technétium 99m servent à explorer le transit digestif (oesophage, estomac, voire intestin grêle et côlon).

Le Technétium 99m est aussi souvent utilisé dans les scintigraphies en double marquage, qui permettent, par soustraction d'image, d'étudier un organe n'ayant pas de traceur spécifique. Ainsi, les glandes parathyroïdes sont visualisées en comparant les images obtenues en utilisant du thallium 201, qui se fixe sur la thyroïde et les parathyroïdes, à celles obtenues en utilisant du Technétium 99m, qui ne se fixe que sur la thyroïde.

Des complexes marqués par un radiométal tel que le technétium 99m sont décrits dans WO 98/31399. Ces complexes sont des substrats du facteur XIIIa utiles pour le diagnostic, notamment du cancer.

Il existe toutefois toujours un besoin de déterminer la localisation précise et/ou la capacité de réponse d'une cellule à un agent cytotoxique particulier. Dans le cas présent, l'intérêt se porte sur les cellules tumorales ou tumeurs sur-exprimant le système de transport des polyamines (système PTS). Il n'existe en effet à ce jour pas de moyen pour déterminer rapidement et de manière non invasive si une tumeur va répondre ou non à un traitement ciblant le système PTS.

La présente invention se propose donc de résoudre ce problème.

La présente invention a ainsi pour premier objet des polyamines conjuguées à l'hydrazinonicotinamide ou polyamines HYNIC de formule (I) suivante : 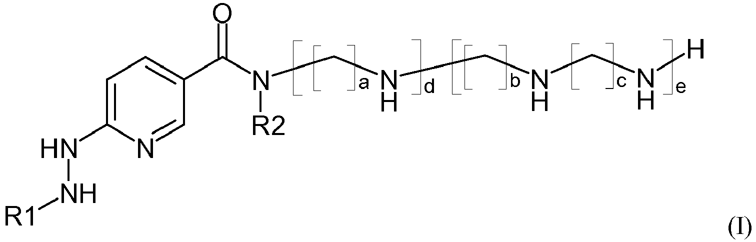 dans laquelle :
- R1 représente un atome d'hydrogène ou un groupement N-protecteur choisi parmi le formyle, l'acétyle, le trifluoroacétyle, le benzoyle, le pivaloyle, le phénylsulfonyle, le benzyle (Bn), le t-butyloxycarbonyle (BOC), le benzyloxycarbonyle (Z), le p-méthoxybenzyloxycarbonyle, le p-nitrobenzyl-oxycarbonyle, le trichloroéthoxycarbonyle (TROC), l'allyloxycarbonyle (Alloc), le 9-fluorénylméthyloxycarbonyle (Fmoc), le trifluoro-acétyle, et les carbamates de benzyle substitués ou non,
- R2 représente un atome d'hydrogène, un groupement C₁₋₆alkyle éventuellement fluoré ou perfluoré, ou un groupement amino-C₁₋₆alkyl,
- a, b et c représentent, indépendamment les uns des autres, un nombre entier de 2 à 5, et
- d et e représentent, indépendamment l'un de l'autre, 0, 1 ou 2, à la condition que d et e ne valent pas tous les deux simultanément 0,
ou un sel pharmaceutiquement acceptable de celles-ci.

Par « groupe protecteur » ou « groupe de protection », on entend désigner, au sens de la présente invention, un groupe qui bloque sélectivement un site réactif dans un composé multifonctionnel de telle sorte qu'une réaction chimique peut être effectuée sélectivement au niveau d'un autre site réactif non protégé dans la signification classiquement associée à celui-ci en chimie de synthèse.

Par « groupe N-protecteur », on entend, au sens de la présente invention, tout substituant qui protège le groupe NH ou NH₂ contre les réactions indésirables tels que les groupes N-protecteur décrits dans Greene, « Protective Groups In Organic synthesis », (John Wiley & Sons, New York (1981)) et Harrison et al. « Compendium of Synthetic Organic Methods », Vols. 1 à 8 (J. Wiley & sons, 1971 à 1996). Les groupes N-protecteurs comprennent les carbamates, les amides, les dérivés N-alkylés, les dérivés amino acétale, les dérivés N-benzylés, les dérivés imine, les dérivés énamine et les dérivés N-hétéroatome. En particulier, le groupe N-protecteur comprend le formyle, l'acétyle, le trifluoroacétyle, le benzoyle, le pivaloyle, le phénylsulfonyle, le benzyle (Bn), le t-butyloxycarbonyle (BOC), le benzyloxycarbonyle (Z), le p-méthoxy benzyloxycarbonyle, le p-nitrobenzyl-oxycarbonyle, le trichloroéthoxycarbonyle (TROC), l'allyloxycarbonyle (Alloc), le 9-fluorénylméthyloxycarbonyle (Fmoc), le trifluoro-acétyle, les carbamates de benzyle et similaires. Il pourra s'agir notamment d'un groupe BOC, Z ou trifluoroacétyle (CF₃CO).

Par groupement « C₁-₆alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, *tert*-butyle, pentyle ou encore hexyle.

Un groupe « C₁-₆alkyle fluoré » est un groupe C₁-₆alkyle tel que défini ci-dessus pour lequel un ou plusieurs atomes d'hydrogène sont remplacés par un atome de fluor.

Un groupe « C₁-₆alkyle perfluoré » est un groupe C₁-₆alkyle tel que défini ci-dessus pour lequel tous les atomes d'hydrogène sont remplacés par un atome de fluor.

Par groupement « amino-C₁-₆alkyl », on entend, au sens de la présente invention, un groupe NH₂-C₁-₆alkyle- avec le groupe C₁-₆alkyle tel que défini précédemment.

Dans la présente invention, on entend désigner par « pharmaceutiquement acceptable » ce qui est utile dans la préparation d'une composition destinée à être administrée à un animal, tel qu'un mammifère, y compris l'homme, qui est généralement sûr, non toxique et ni biologiquement ni autrement non souhaitable et qui est acceptable pour une utilisation vétérinaire, de même que pharmaceutique ou diagnostique humaine.

On entend désigner par « sels pharmaceutiquement acceptables » d'un composé, des sels qui sont pharmaceutiquement acceptables, comme défini ici, et qui possèdent l'activité pharmacologique ou diagnostique souhaitée du composé parent. De tels sels comprennent :
(1) les hydrates et les solvates,
(2) les sels d'addition d'acide pharmaceutiquement acceptable formés avec des acides inorganiques ou organiques pharmaceutiquement acceptables tels que l'acide chlorhydrique ou l'acide bromhydrique, ou
(3) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable.

De préférence, il s'agira de sels d'addition d'acide, tel qu'un sel d'addition d'acide chlorhydrique.

Dans la définition de R1, le groupe N-protecteur sera plus particulièrement un groupe -protecteur clivable en milieu acide tel qu'un groupe BOC ou trifluoroacétyle (CF₃CO).

R1 représentera plus particulièrement un atome d'hydrogène ou un groupement *tert*-butyloxycarbonyle (*t*BuOCO) ou trifluoroacétyle (CF₃CO).

R2 représentera notamment un atome d'hydrogène ou un groupement C₁₋₆alkyle tel que méthyle.

Plus particulièrement, a, b et c représenteront, indépendamment les uns des autres, 3 ou 4.

De préférence, d et e représentent 1 ou 2, et avantageusement 1.

Selon un autre mode de réalisation de l'invention, e représente 2 et d représente 0.

Selon encore un autre mode de réalisation de l'invention, e représentera _{[GS1]}1, de préférence 1, et d représentera 0, 1 ou 2, de préférence 1 ou 2.

Il pourra s'agir plus particulièrement d'un composé de formule (I) pour lequel :
- R1 = R2 = H, a = 4, d = 1 et e = 0,
- R1 = R2 = H, b = 3, c = 4, d = 0 et e = 1,
- R1 = R2 = H, a = 3, b = 4, c = 3, d = 1 et e = 1,
- R1 = R2 = H, a = 3, b = 4, c = 3, d = 2 et e = 1,
- R1 = CF₃CO, R2 = H, a = 3, b = 4, c = 3, d = 1 et e = 1,
- R1 = *t*BuOCO, R2 = H, a = 3, b = 4, c = 3, d = 1 et e = 1, ou
- R1 =H, R2 = CH₃, b = 3, c = 4, d = 0 et e = 1,
ou d'un sel pharmaceutiquement acceptable de celui-ci.
Avantageusement il s'agira du composé de formule I pour lequel :
- R1 = R2 = H, a = 3, b = 4, c = 3, d = 1 et e = 1,
- R1 = R2 = H, a = 3, b = 4, c = 3, d = 2 et e = 1,
- R1 = CF₃CO, R2 = H, a = 3, b = 4, c = 3, d = 1 et e = 1, ou
- R1 = *t*BuOCO, R2 = H, a = 3, b = 4, c = 3, d = 1 et e = 1,
ou d'un sel pharmaceutiquement acceptable de celui-ci.

De tels composés ne sont pas utiles en tant que tels mais permettent de complexer le technétium 99m avec d'autres ligands tels que la tricine. Le complexe ainsi formé sera utile comme radio-traceur pour une utilisation en imagerie de scintigraphie pour mettre en évidence des tumeurs cancéreuses exprimant le système de transport des polyamines.

La présente invention a donc pour deuxième objet un complexe de formule (II) suivante : 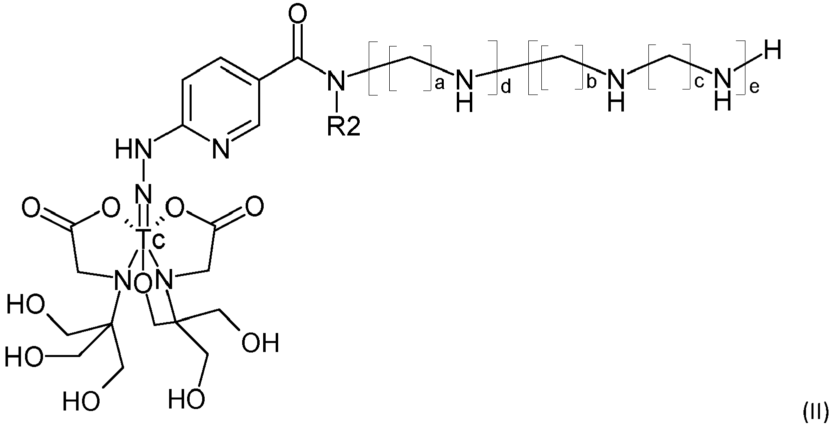 dans laquelle le technétium (Tc) est présent sous forme de son isotope 99m et R2, a, b, c, d et e sont tels que définis précédemment,
ou un sel pharmaceutiquement acceptable de celui-ci.

R2 représentera notamment un atome d'hydrogène ou un groupement C₁₋₆alkyle tel que méthyle.

Plus particulièrement, a, b et c représenteront, indépendamment les uns des autres, 3 ou 4.

De préférence, d et e représentent 1 ou 2, et avantageusement 1.

En effet, il est préférable que le motif polyamine des composés de la présente invention comporte au moins trois, et avantageusement trois, azotes basiques afin que les composés de formule (II) soient bien pris en charge par le système de transport des polyamines et donc que le marquage soit suffisant pour permettre la mise en évidence des tumeurs exprimant ce système de transport des polyamines.

Selon un autre mode de réalisation de l'invention, e représente 2 et d représente 0.

Selon encore un autre mode de réalisation de l'invention, e représentera 1, de préférence 1, et d représentera 0, 1 ou 2, de préférence 1 ou 2.

Il pourra s'agir plus particulièrement d'un composé de formule (II) pour lequel :
- R2 = H, a = 4, d = 1 et e = 0,
- R2 =H, b = 3, c = 4, d = 0 et e = 1,
- R2=H, a = 3, b = 4, c = 3, d = 1 et e = 1,
- R2 = H, a = 3, b = 4, c = 3, d = 2 et e = 1, ou
- R2 = CH₃, b = 3, c = 4, d = 0 et e = 1,
ou d'un sel pharmaceutiquement acceptable de celui-ci.
Avantageusement, il s'agira d'un composé de formule II pour lequel :
- R2 = H, a = 3, b = 4, c = 3, d = 1 et e = 1, ou
- R2 = H, a = 3, b = 4, c = 3, d = 2 et e = 1,
ou d'un sel pharmaceutiquement acceptable de celui-ci.

La présente invention a pour troisième objet un complexe de formule (II) tel que défini ci-dessus ou d'un sel pharmaceutiquement acceptable de celui-ci pour son utilisation en tant que sonde de diagnostic pour imagerie médicale, notamment par scintigraphie, plus particulièrement pour mettre en évidence une tumeur cancéreuse exprimant le système de transport des polyamines *in vivo* ou *in vitro,* et notamment *in vivo.*

La scintigraphie est une méthode d'imagerie médicale utilisant des composés marqués avec des isotopes radioactifs. Ces composés radio-marqués sont administrés à un animal, tel qu'un mammifère, y compris l'homme, et permettent de produire une image médicale par la détection des rayonnements émis par les isotopes radioactifs. Selon le composé marqué utilisé, il sera possible de visualiser différentes parties du corps, selon la cible du composé marqué.

La présente invention a pour quatrième objet un complexe de formule (II) tel que défini ci-dessus ou un sel pharmaceutiquement acceptable de celui-ci pour son utilisation dans le diagnostic, plus particulièrement *in vivo,* d'une tumeur cancéreuse exprimant le système de transport des polyamines, notamment par imagerie médicale, telle que par scintigraphie.

En effet, le complexe du Technétium 99m de formule (II) reconnaît les cellules tumorales en profitant de leur capacité d'internaliser les polyamines naturelles dont elles ont besoin pour leur métabolisme.

Un tel complexe de Technétium 99m, injecté au patient, permet de mettre en évidence l'existence d'un foyer tumoral car le complexe est distribué préférentiellement dans la tumeur.

Cette approche permet de sélectionner les patients dont la tumeur exprime bien le transporteur de polyamine ou système PTS. Elle est déterminante pour pouvoir traiter lesdits patients avec un produit anticancéreux qui serait lui-même vectorisé par les polyamines naturelles, ciblant ainsi les cellules tumorales par rapport aux cellules saines. Cette sélection de patients permet d'avoir un taux de réponse plus favorable, et de ne pas traiter les patients non-répondeurs.

La présente invention concerne également un complexe de formule (II) tel que défini ci-dessus utilisé pour la préparation d'une composition diagnostique destinée à la mise en évidence d'une tumeur cancéreuse exprimant le système de transport des polyamines, plus particulièrement *in vivo,* notamment par imagerie médicale, telle que par scintigraphie.

La présente invention concerne également une méthode de mise en évidence (ou diagnostique) d'une tumeur cancéreuse exprimant le système de transport des polyamines comprenant l'administration à une personne en ayant besoin d'une quantité suffisante d'un complexe de formule (II) tel que défini ci-dessus. Cette administration est suivie d'une étape de détection de la radioactivité émise par le Technétium 99m, notamment par imagerie de scintigraphie afin de visualiser la tumeur.

La présente invention a pour cinquième objet une composition comprenant au moins un composé de formule (I) tel que défini précédemment ou un sel pharmaceutiquement acceptable de celui-ci et au moins un excipient pharmaceutiquement acceptable.

L'excipient pharmaceutiquement acceptable sera plus particulièrement un excipient utilisé dans les compositions administrées par voie parentérale.

Ces compositions comprendront notamment des suspensions aqueuses, des solutions salines isotoniques ou des solutions stériles et injectables qui contiennent des agents de dispersion et/ou des agents mouillants pharmacologiquement compatibles.

Les composés de formule (I) peuvent être présents à des doses comprises entre 0,01 mg et 1000 mg. La dose peut être avantageusement comprise entre 5 mg et 500 mg, et notamment entre 10 mg et 200 mg. Il peut être nécessaire d'utiliser des doses sortant de ces gammes ce dont l'homme du métier peut se rendre compte lui-même.

La présente invention a pour sixième objet une composition diagnostique comprenant au moins un complexe de formule (II) selon l'invention ou un sel pharmaceutiquement acceptable de celui-ci et au moins un excipient pharmaceutiquement acceptable.

Par « composition diagnostique », on entend, au sens de la présente invention, une composition destinée à être administrée à un animal tel qu'un mammifère, y compris l'homme, en vue de réaliser un diagnostic, et plus particulièrement un diagnostic *in vivo,* notamment par imagerie médicale telle que par scintigraphie. Dans le cadre de la présente invention, il s'agira plus particulièrement de permettre la mise en évidence de tumeurs cancéreuses exprimant le système de transport des polyamines.

Le technétium présent dans une telle composition sous forme de complexe de formule (II) sera totalement ou partiellement (c'est-à-dire au moins 80 %, de préférence au moins 90 %, encore préférentiellement de plus de 90 %, encore plus préférentiellement au moins de 95 % et toujours préférentiellement de près de 100 %) sous forme d'isotope 99m.

Les compositions diagnostiques selon l'invention peuvent être formulées pour tous types d'administrations souhaitables, préférentiellement par voie parentérale, notamment par intraveineuse.

L'ingrédient actif peut être administré sous formes unitaires d'administration, en mélange avec des supports pharmaceutiques classiques, aux animaux ou aux êtres humains.

Pour une administration intraveineuse, on utilisera notamment des suspensions aqueuses, des solutions salines isotoniques ou des solutions stériles et injectables qui contiennent des agents de dispersion et/ou des agents mouillants pharmacologiquement compatibles.

Les complexes de formule (II) en tant qu'agent de diagnostic peuvent être utilisés à des doses comprises entre 5 mg et 500 mg. Il peut être nécessaire d'utiliser des doses sortant de ces gammes ce dont l'homme du métier peut se rendre compte lui-même.

Une telle composition diagnostique est utile pour le diagnostic d'une tumeur cancéreuse exprimant le système de transport des polyamines, notamment par imagerie médicale, telle que par scintigraphie.

La présente invention a pour septième objet un procédé de préparation d'une composition diagnostique telle que définie ci-dessus comprenant le mélange d'une composition comprenant au moins un composé de formule (I) ou un sel pharmaceutiquement acceptable de celui-ci tel que défini ci-dessus avec un sel de pertechnétate-99m, au moins un agent réducteur et de la tricine.

Le procédé est réalisé de préférence à température ambiante, c'est-à-dire à une température comprise entre 15 et 40°C, notamment entre 20 et 35°C, en particulier à environ 25°C.

Le sel de pertechnétate sera de préférence un sel de métal alcalin tel qu'un sel de sodium. Un tel sel sera plus particulièrement utilisé en solution dans une solution physiologique. Une telle solution est obtenue à l'aide d'un générateur à molybdène 99 comme décrit précédemment.

L'agent réducteur pourra être un mélange de fluorure d'étain et d'acide ascorbique permettant de réduire le Technétium du degré d'oxydation +VII au degré +III pour permettre sa complexation avec le composé de formule (I) et la tricine.

La tricine sert de ligand du Technétium 99m. Elle répond à la formule suivante :

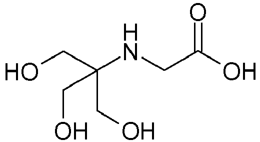

Par exemple, pour 1,5mg de composé de formule (I), il pourra être utilisé 24 mg de tricine, 80 µg de fluorure d'étain et 0,5 mg d'acide ascorbique.

Une telle composition est préparée de manière extemporanée, c'est-à-dire juste avant son utilisation.

La présente invention a donc pour huitième objet un kit comprenant :
(1) une composition comprenant au moins un composé de formule (I) tel que défini précédemment ou un sel pharmaceutiquement acceptable de celui-ci et au moins un excipient pharmaceutiquement acceptable,
(2) de la tricine, et
(3) un agent réducteur.

Ce kit pourra comprendre en outre les instructions nécessaires pour utiliser ce kit, notamment dans le diagnostic de tumeurs cancéreuses.

L'agent réducteur pourra être un mélange de fluorure d'étain et d'acide ascorbique.

Par exemple, pour 1,5 mg de composé de formule (I) présent dans la composition (1), le kit pourra contenir 24 mg de tricine, 80 µg de fluorure d'étain et 0,5 mg d'acide ascorbique.

La présente invention a pour neuvième objet un procédé de préparation d'un composé de formule (I) tel que défini ci-dessus ou d'un sel pharmaceutiquement acceptable de celui-ci comprenant les étapes successives suivantes :
(a) réaction de l'acide 6-halogéno-nicotinique avec une polyamine protégée de formule (III) suivante : 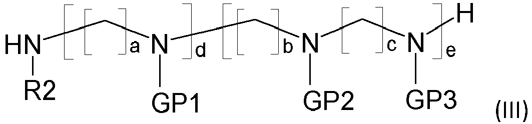 pour laquelle R2, a, b, c, d et e sont tels que définis précédemment et GP1, GP2 et GP3, identiques ou différents, représentent chacun un groupement N-protecteur, pour donner un composé de formule (IV) suivante : 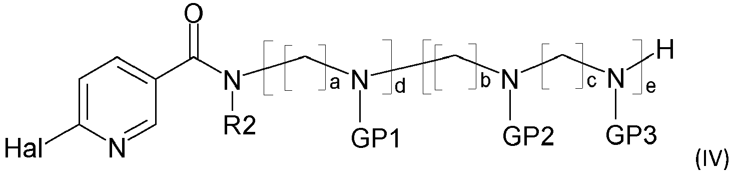 pour laquelle R2, a, b, c, d, e, GP1, GP2 et GP3 sont tels que définis précédemment et Hal représente un atome d'halogène,
(b) réaction du composé de formule (IV) obtenu à l'étape (a) précédente avec de l'hydrazine pour donner un composé de formule (V) suivante : 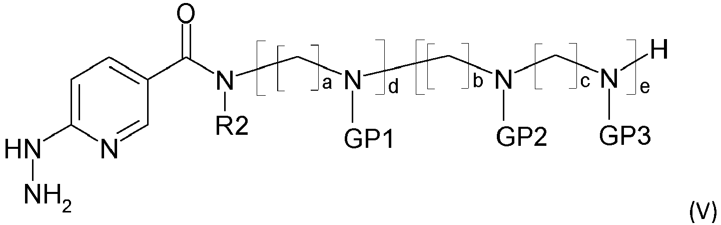 pour laquelle R2, a, b, c, d, e, GP1, GP2 et GP3 sont tels que définis précédemment,
(c) éventuellement protection par un groupement N-protecteur de la fonction hydrazine du composé de formule (V) obtenu à l'étape (b) précédente pour donner un composé de formule (VI) suivante : 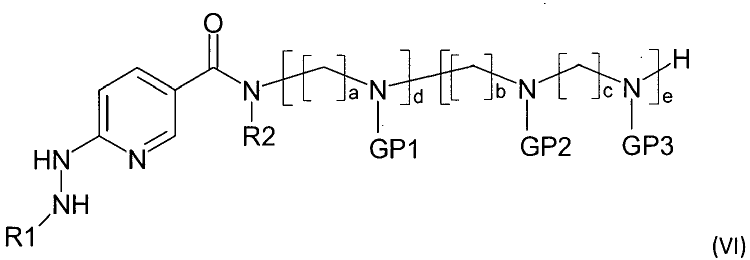 pour laquelle R2, a, b, c, d, e, GP1, GP2 et GP3 sont tels que définis précédemment et R1 représente un groupement N-protecteur tel que défini ci-dessus,
(d) déprotection des fonctions amines protégées par les groupements GP1, GP2 et GP3 dans le composé de formule (V) ou (VI) obtenu à l'étape (b) ou (c) précédente pour donner un composé de formule (I) selon l'invention,
(e) éventuellement salification du composé de formule (I) obtenu à l'étape (d) précédente pour obtenir un sel pharmaceutiquement acceptable de celui-ci, et
(f) séparation du composé de formule (I) ou de son sel pharmaceutiquement acceptable obtenu à l'étape précédente du milieu réactionnel.

Par « halogéno » ou « halogène », on entend, au sens de la présente invention, un atome d'iode, de fluor, de brome ou de chlore. Il s'agit notamment d'un atome de chlore.

### Etape (a) :

L'acide 6-halogéno-nicotinique sera de préférence de l'acide 6-chloro-nicotinique, composé disponible dans le commerce.

Dans cette étape, les groupements GP1, GP2 et GP3 peuvent représenter un groupe BOC ou Z. De préférence, ces trois groupements protecteurs sont identiques.

Les polyamines non protégées sont disponibles dans le commerce et peuvent être protégées par des techniques bien connues de l'homme du métier permettant d'avoir accès facilement aux composés de formule (III). Un exemple de dérivé polyamine, la spermine protégée par trois groupements BOC, est décrit dans Tetrahedron Lett. 1998, 39, 439.

La fonction amide du composé de formule (IV) peut être formée par couplage peptidique entre la fonction acide carboxylique de l'acide 6-halogéno-nicotinique et la fonction amine libre de la polyamine protégée de formule (III).

Le couplage peptidique pourra être réalisé en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tetraméthyluronium hexafluorophosphate (HBTU), le 2-(1H-benzotriazole-1-yl)-1,1,3,3-tetraméthyluronium tetrafluoroborate (TBTU) ou encore le O-(7-azobenzotriazol-1-yl)-1,1,3,3-tétraméthyluronium hexafluorophosphate (HATU), éventuellement associé à un auxiliaire de couplage tel que le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HAt) ou le N-hydroxysylfosuccinimide (sulfo NHS). En particulier, le couplage pourra être réalisé en présence de TBTU.

Le couplage pourra en outre être réalisé en présence d'une base telle que la triéthylamine. Un solvant inerte tel que l'acétonitrile pourra être utilisé.

### Etape (b) :

L'hydrazine utilisée sera plus particulièrement de l'hydrate d'hydrazine, disponible dans le commerce.

La réaction pourra être réalisée à température élevée, notamment au reflux de l'hyrazine, en particulier à environ 100°C.

### Etape (c) :

Cette étape de protection peut être effectuée par des techniques bien connues de l'homme du métier.

Lorsque le groupe N-protecteur est un groupe BOC, elle peut être réalisée par réaction avec du *tert*-butyl carbonate en présence d'une base telle que de la triéthylamine. Une telle réaction peut être réalisée à température ambiante, notamment dans un solvant tel que le THF.

### Etape (d) :

L'étape de déprotection peut être réalisée par des techniques bien connues de l'homme du métier.

Lorsque les groupements protecteurs sont des groupements BOC, la déprotection pourra être réalisée en milieu acide, notamment en présence d'acide chlorhydrique ou d'acide trifluoroacétique. Une telle réaction pourra être réalisée à température ambiante, notamment dans un solvant tel que le dioxane ou l'isopropanol.

Lorsque les groupements protecteurs sont des groupements Z, la déprotection pourra être réalisée par hydrogénation sous atmosphère d'hydrogène en présence d'un catalyseur d'hydrogénation tel que Pd/C. Une telle réaction pourra être réalisée en milieu alcoolique, notamment dans le méthanol ou l'éthanol. Une protection par des groupements Z sera préférée lorsque R1 représentera un groupement N-protecteur clivable en milieu acide. En effet, cela permettra de déprotéger les groupements GP1, GP2 et GP3 sans déprotéger le groupement R1.

### Etape (e) :

Cette étape pourra être réalisée par réaction du composé de formule (I) obtenu à l'étape (d) avec un acide ou une base pharmaceutiquement acceptable. Il s'agira de préférence d'un acide pharmaceutiquement acceptable tel que l'acide chlorhydrique.

Selon la nature des groupements protecteurs GP1, GP2 et GP3 et le sel pharmaceutiquement acceptable désiré (et notamment quand GP1 = GP2 = GP3 = BOC et le sel est le chlorhydrate), il peut être envisagé d'effectuer les étapes (d) et (e) de manière « one-pot », c'est-à-dire dans un même réacteur, sans isoler l'intermédiaire de synthèse entre les deux étapes, et notamment en utilisant les mêmes réactifs (notamment le même acide pour déprotéger les groupements GP1, GP2 et GP3 et pour former le sel pharmaceutiquement acceptable).

### Etape (f) :

Le composé obtenu pourra être séparé du milieu réactionnel par des méthodes bien connues de l'homme du métier, comme par exemple par extraction, évaporation du solvant ou encore par précipitation et filtration.

Le composé pourra être purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

La présente invention a pour neuvième objet un procédé de préparation d'un composé de formule (I) tel que défini ci-dessus pour lequel R1 ≠ H ou d'un sel pharmaceutiquement acceptable de celui-ci comprenant les étapes successives suivantes :
(i) protection par un groupement N-protecteur de la fonction hydrazine de l'acide 6-hydrazinyl-nicotinique pour donner un composé de formule (VII) suivante : 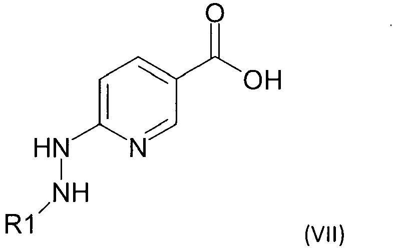 pour laquelle R1 représente un groupement N-protecteur tel que défini ci-dessus,
(ii) réaction d'un composé de formule (VII) obtenu à l'étape (i) précédente avec une polyamine protégée de formule (III) telle que définie précédemment pour donner un composé de formule (VI) tel que défini ci-dessus,
(iii) déprotection des fonctions amines protégées par les groupements GP1, GP2 et GP3 dans le composé de formule (VI) obtenu à l'étape (ii) précédente pour donner un composé de formule (I) selon l'invention pour lequel R1 ≠ H,
(iv) éventuellement salification du composé de formule (I) obtenu à l'étape (iii) précédente pour obtenir un sel pharmaceutiquement acceptable de celui-ci, et
(v) séparation du composé de formule (I) ou de son sel pharmaceutiquement acceptable obtenu à l'étape précédente du milieu réactionnel.

### Etape (i) :

L'acide 6-hydrazinyl-nicotinique répond à la formule suivante :

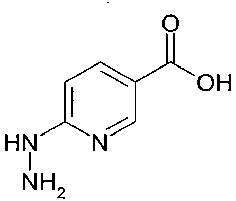

Il peut être obtenu par réaction d'un acide 6-halogéno-nicotinique, tel que l'acide 6-chloro-nicotinique, avec de l'hydrazine, et plus particulièrement de l'hydrate d'hydrazine. Une telle réaction pourra être réalisée à température élevée, notamment au reflux de l'hyrazine, en particulier à environ 100°C.

La réaction de protection de la fonction hydrazine par un groupement N-protecteur se fait par des méthodes bien connues de l'homme du métier, notamment par l'une des méthodes décrites à l'étape (c) précédente.

Lorsque le groupement protecteur est le trifluoroacétyle, la réaction de protection peut être effectuée en présence du chlorure de l'acide trifluoroacétique ou bien par protection par un groupement Boc (voir étape (c)) notamment comme décrit dans J. Med. Chem. 2007, 50, 1418-1422.

### Etape (ii) :

Cette étape peut être réalisée dans les mêmes conditions que celles de l'étape (a) décrite précédemment.
Etape (iii) : voir étape (d) précédente.
Etape (iv) : voir étape (e) précédente.
Etape (v) : voir étape (f) précédente.

La présente invention a pour dixième objet un procédé de préparation d'un composé de formule (II) tel que défini ci-dessus ou d'un sel pharmaceutiquement acceptable de celui-ci comprenant le mélange d'un composé de formule (I) tel que défini ci-dessus ou d'un sel pharmaceutiquement acceptable de celui-ci avec un sel de pertechnétate-99m, au moins un agent réducteur et de la tricine.

Le procédé est réalisé de préférence à température ambiante, c'est-à-dire à une température comprise entre 15 et 40°C, notamment entre 20 et 35°C, en particulier à environ 25°C.

Ce procédé sera réalisé avantageusement dans un milieu aqueux, notamment pharmaceutiquement acceptable.

Le sel de pertechnétate sera de préférence un sel de métal alcalin tel qu'un sel de sodium.

L'agent réducteur pourra être un mélange de fluorure d'étain et d'acide ascorbique permettant de réduire le Technétium du degré d'oxydation +VII au degré +III pour permettre sa complexation avec le composé de formule (I) et la tricine.

La tricine sert de ligand du Technétium 99m. Elle répond à la formule suivante :

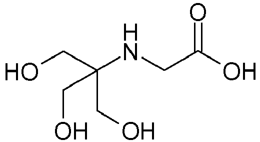

Par exemple, pour 1,5 mg de composé de formule (I), il pourra être utilisé 24 mg de tricine, 80 µg de fluorure d'étain et 0,5 mg d'acide ascorbique.

La présente invention sera mieux comprise à la lumière des exemples non limitatifs qui suivent.

### FIGURES

La **Figure 1** représente le pourcentage d'incorporation d'un complexe de formule (II) dans des cellules B16/F10 et A549 incubées avec différentes concentrations de complexe de formule (II) et de spermine.
La **Figure 2** représente l'image TEMP d'une souris traitée selon l'exemple 30 1).
Les **Figure 3a** et **3b** représentent des images TEMP d'une souris traitée selon l'exemple 31 avec un composé de formule (II) pour lequel R2 = H, a = 3, b = 4, c = 3, d = e = 1 obtenu à partir d'un composé de formule (I) pour lequel R1 = H ou CF₃CO respectivement.

### EXEMPLES

### Exemple 1 : Préparation de l'ester tert-butylique de l'acide {4-[(6-chloro-pyridine-3-carbonyl)-amino]-butyl}-carbamique

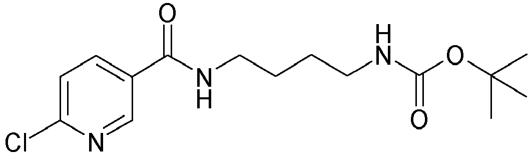

Au mélange de 2 g d'acide 6-chloronicotinique et de 2,4 g de N-BOC-1,4-diaminobutane en solution dans 100 mL d'acétonitrile à température ambiante sous agitation et en présence de 2,1 mL de triéthylamine sont ajoutés en une fois 4,1 g de TBTU. Le mélange est laissé en contact à cette température environ 5 heures. Le milieu réactionnel est hydrolysé avec 300 mL de solution aqueuse d'acide chlorhydrique 0,5 M puis extrait avec de l'acétate d'éthyle (3x100 mL). Après décantation, séchage sur sulfate de sodium anhydre et filtration, le solvant est évaporé sous pression réduite. Le résidu obtenu est purifié par flash chromatographie SiO₂ avec un gradient allant de 100 % de CH₂Cl₂ au mélange CH₂Cl₂/Méthanol/NH₄OH (80/18/2) pour obtenir 2,1 g de solide couleur crème après évaporation des fractions concernées. (Rdt : 50 %). CCM SiO₂ : CH₂Cl₂/Méthanol/NH₄OH (90/9/1). Rf : 0,63.

### Exemple 2 : Préparation de l'ester tert-butylique de l'acide {4-[(6-hydrazino-pyridine-3-carbonyl)-amino]-butyl}-carbamique.

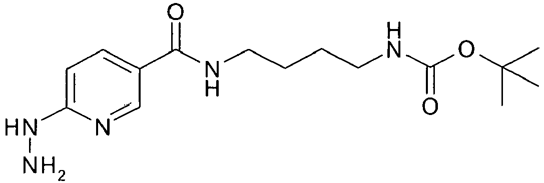

A 2,1 g de l'ester *tert*-butylique de l'acide {4-[(6-chloro-pyridine-3-carbonyl)-amino]-butyl}-carbamique sont ajoutés 30 mL d'hydrate d'hydrazine. Le mélange obtenu est porté au reflux pendant environ 5 heures. Après hydrolyse dans 300 mL d'eau, le milieu est extrait à l'acétate d'éthyle (3x100 mL). Les phases organiques sont lavées avec une solution saturée en NaCl, puis séchées sur sulfate de sodium anhydre. Après filtration, le solvant est évaporé sous pression réduite. Le résidu obtenu est purifié par flash chromatographie SiO₂ avec un gradient allant de CH₂Cl₂ 100 % à CH₂C₂/méthanol 80/20. Une purification complémentaire par HPLC préparative sur colonne Sunfire C18 OBD de Waters 10 µ, 19x250 mm, a été réalisée avec comme phase mobile un gradient allant de HCl 5 mmol à acétonitrile/HCl 5 mmol 50/50 pour obtenir après lyophilisation des fractions concernées 210 mg du composé sous sa forme chlorhydrate obtenu sous forme de poudre blanche. (Rdt : 10 %).
C₁₅H₂₅N₅O₃: 323,398
Analyse HPLC sur colonne Waters X-Brigde C18, 5 µ, 4,6x250 mm
Élution : Acétonitrile/Tampon KH₂PO₄ 6,8 g/L pH 4 (2/98), débit 1 mL/minute,
λ : 220 nm. Temps rétention : 3,43 min.

### Exemple 3 : Préparation de {N-(4-Amino-butyl)-6-hydrazino-nicotinamide, composé de formule (I) (R1 = R2 = H, a = 4, d = 1, e = 0).

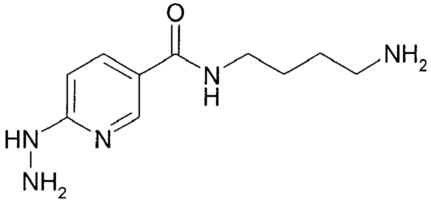

0,21 g du chlorhydrate de l'ester *tert*-butylique de l'acide {4-[(6-hydrazino-pyridine-3-carbonyl)-amino]-butyl}-carbamique sont dissous dans 10 mL de solution 4M d'acide chlorhydrique dans le dioxane. Le milieu réactionnel obtenu est laissé sous agitation 6 heures à température ambiante. Le précipité résultant est filtré sous vide, rincé à l'éther éthylique et séché sous vide. Une purification par HPLC préparative sur colonne Sunfire C18 OBD de Waters 10 µ, 19x250 mm a été réalisée avec comme phase mobile un gradient allant de HCl 5 mmol à acétonitrile/HCl 5 mmol 50/50 pour obtenir après lyophilisation des fractions concernées 55 mg de chlorhydrate du composé sous forme de poudre de couleur crème. (Rdt : 28 %).
Pf : 275°C.
Analyse HPLC sur colonne Waters Atlantis HILIC, 5 µ, 4,6x150 mm
Élution : Acétonitrile/eau/formiate d'ammonium 750/250/0,63 g pH 5, débit 1 mL/minute,
λ : 220 nm. Temps rétention : 14,64 min.
C₁₀H₁₇N₅O : 223,28 ; Chlorhydrate : C₁₀H₁₇N₅O, 2 HCl : 296,.200 - Masse (ESI+400°C) : 224,2 (M+H)
¹H-RMN (400MHz, DMSO-d6) δ = 8.57 (1H, s, H-2 Ar), 8.13 (1H, d, j = 8.8 Hz, H-4 Ar), 6.94 (1H, d, j = 8.8 Hz, H-5 Ar), 3.28 (2H, m, CH₂NHCO), 2.82 (2H, m, CH₂NH₂), 2.54 (4H, m, CH2-CH2).

### Exemple 4 : Préparation de l'ester benzylique de l'acide [4-(benzyloxycarbonyl-{3-[(6-chloro-pyridine-3-carbonyl)-amino]-propyl}-amino)-butyl]-carbamique

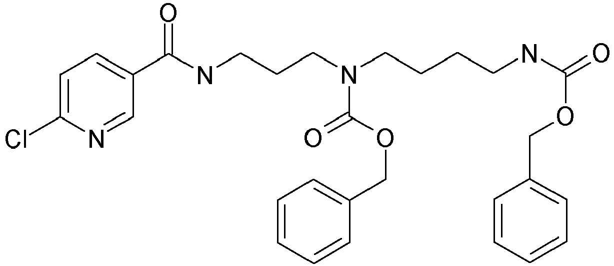

Au mélange de 0,23 g d'acide 6-chloronicotinique et de 0,77 g de l'ester benzylique de l'acide {4-[(3-amino-propyl)-benzyloxycarbonyl-amino]-butyl}-carbamique (J. Med. Chem. 1997, 40, 3842-3850) en solution dans 30 mL d'acétonitrile à température ambiante sous agitation et en présence de 0,3 mL de triéthylamine sont ajoutés en une fois 0,6 g de TBTU. Le mélange est laissé en contact à cette température environ 6 heures. Le milieu réactionnel est hydrolysé avec 200 mL de solution aqueuse d'acide chlorhydrique 0,5 M puis extrait avec de l'acétate d'éthyle (3x10 mL). Après décantation, séchage sur sulfate de sodium anhydre et filtration, le solvant est évaporé sous pression réduite. Le résidu obtenu est purifié par flash chromatographie SiO₂ avec un gradient allant de 100 % de CH₂Cl₂ au mélange CH₂Cl₂/Méthanol (97/3) pour obtenir 0,52 g d'huile incolore après évaporation des fractions concernées. (Rdt : 50 %).
CCM SiO₂ : CH₂Cl₂/Méthanol (95/5) - Rf: 0.44

### Exemple 5 : Préparation de l'ester benzylique de l'acide [4-(benzyloxycarbonyl-{3-[(6-hydrazino-pyridine-3-carbonyl)-amino]-propyl}-amino)-butyl]-carbamique

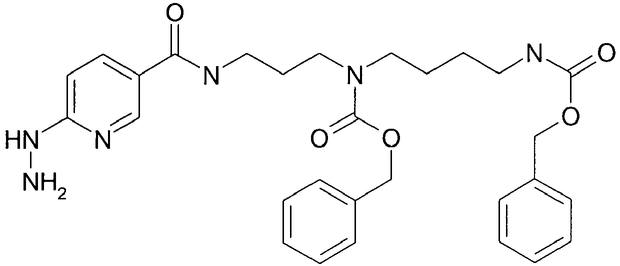

A 0,52 g de l'ester benzylique de l'acide [4-(benzyloxycarbonyl-{3-[(6-chloro-pyridine-3-carbonyl)-amino]-propyl}-amino)-butyl]-carbamique sont ajoutés 20 mL d'hydrate d'hydrazine. Le mélange obtenu est porté au reflux pendant environ 7 heures. Après hydrolyse dans 300 mL d'eau, le milieu est extrait à l'acétate d'éthyle (3x100 mL). Les phases organiques sont lavées avec une solution saturée en NaCl puis séchées sur sulfate de sodium anhydre. Après filtration, le solvant est évaporé sous pression réduite. Le résidu obtenu est purifié par flash chromatographie SiO₂ avec un mélange CH₂Cl₂/méthanol 97/3. Les fractions concernées sont évaporées sous pression réduite pour obtenir 350 mg du composé sous forme d'huile jaune. (Rdt : 68 %).
CCM SiO₂ : CH₂Cl₂/Méthanol/NH₄OH (90/10/1) - Rf: 0.41
C₂₉H₃₆N₆O₅: 548,647

### Exemple 6 : Préparation du N-[3-(4-amino-butylamino)-propyl]-6-hydrazino-nicotinamide, composé de formule (I) (R1= R2 = H, d = 0, b = 3, c = 4, e = 1).

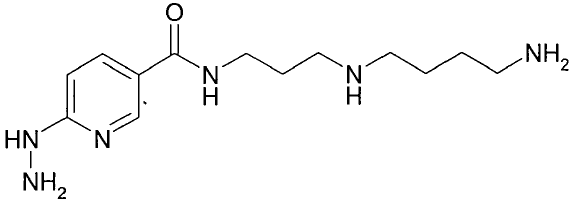

0,35 g de l'ester benzylique de l'acide [4-(benzyloxycarbonyl-{3-[(6-hydrazino-pyridine-3-carbonyl)-amino]-propyl}-amino)-butyl]-carbamique en solution dans 20 mL de méthanol en présence de Pd/10 % est agité violemment sous atmosphère d'hydrogène à température ambiante pendant 5 heures. Le catalyseur est filtré sous vide, rincé au méthanol. Le filtrat est évaporé sous pression réduite pour obtenir un résidu huileux. Le tétra-chlorhydrate est précipité par addition de 4 équivalents d'une solution 4 M d'acide chlorhydrique dans le dioxane à une solution du résidu obtenu dans l'éther éthylique. Le précipité orange est filtré, rincé à l'éther éthylique puis séché sous vide pour donner 145 mg de sel. (Rdt : 53 %)
Pf : 183°C
C₁₃H₂₄N₆O : 280,376 ; sel C₁₃H₂₄N₆O, 4 HCl : 426,220 - Masse (ESI+ 400°C) : 281,2 (M+H).
Pf : 183°C
¹H-RMN (400MHz, DMSO-d6) δ = 8.64 (1H, s, H-2 Ar), 8.17 (1H, d, j = 8.8 Hz, H-4 Ar), 6.95 (1H, d, j = 8.8 Hz, H-5 Ar), 3.33 (2H, m, CH₂NHCO), 2.91 (4H, m, CH₂NH₂), 2.80 (2H, m, CH2-NH2), 1.66-1.90 (6H, m, H2C-CH2).

### Exemple 7 : Préparation de l'ester tert-butylique de l'acide (3-tert-butoxycarbonylamino-propyl)-[4-(tert-butoxycarbonyl-{3-[(6-chloro-pyridine-3-carbonyl)-amino]-propyl}-amino)-butyl]-carbamique

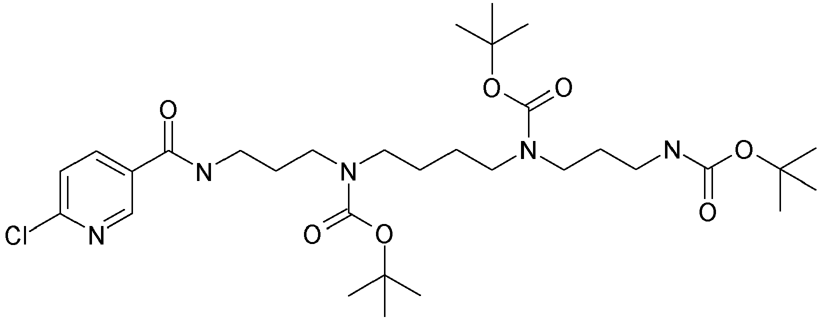

Au mélange de 0,31 g d'acide 6-chloronicotinique et de 1 g de Tri-BOC-spermine (d'après FR 2919287) en solution dans 40 mL d'acétonitrile à température ambiante sous agitation et en présence de 0,3 L de triéthylamine sont ajoutés en une fois 0,4 g de TBTU. Le mélange est laissé en contact à cette température environ 1/2 heure. Le milieu réactionnel est hydrolysé avec 100 mL de solution aqueuse d'acide chlorhydrique 0,5 M, puis extrait avec de l'acétate d'éthyle (3x50 mL). Après décantation, séchage sur sulfate de sodium anhydre et filtration, le solvant est évaporé sous pression réduite. Le résidu obtenu est purifié par flash chromatographie SiO₂ avec un gradient allant de 100 % d'heptane à 100 % d'acétate d'éthyle pour obtenir 0,79 g d'huile incolore après évaporation des fractions concernées. (Rdt : 62 %).
CCM SiO₂ : CH₂Cl₂/Méthanol/NH₄OH (90/9/1). Rf : 0.56

### Exemple 8 : Préparation de l'ester tert-butylique de l'acide (3-tert-butoxycarbonylamino-propyl)-[4-(tert-butoxycarbonyl-{3-[(6-hydrazino-pyridine-3-carbonyl)-amino]-propyl}-amino)-butyl]-carbamique

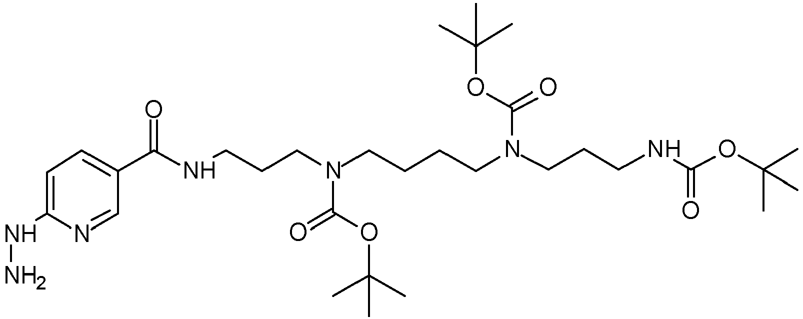

A 0,79 g de l'ester *tert*-butylique de l'acide (3-*tert*-butoxycarbonylamino-propyl)-[4-(*tert*-butoxycarbonyl-{3-[(6-chloro-pyridine-3-carbonyl)-amino]-propyl}-amino)-butyl]-carbamique sont ajoutés 30 mL d'hydrate d'hydrazine. Le mélange obtenu est porté au reflux pendant environ 6 heures. Après hydrolyse dans 500 mL d'eau, le milieu est extrait à l'acétate d'éthyle (3x100 mL). Les phases organiques sont lavées avec une solution saturée en NaCl puis séchées sur sulfate de sodium anhydre. Après filtration, le solvant est évaporé sous pression réduite pour donner 0,74 g de résidu huileux verdâtre. (Rdt : 94%).
CCM SiO₂ : CH₂Cl₂/methanol/NH₄OH (90/9/1). Rf: 0,46.
C₃₁H₅₅N₇O₇ : 637,827 - Masse (ESI+ 400°C) : 638,4 (M+H).

### Exemple 9 : Préparation du N-{3-[4-(3-amino-propylamino)-butyl amino]-propyl}-6-hydrazino-nicotinamide, composé de formule (I) (R1 = R2 = H, a = 3, b = 4, c = 3, d = e = 1).

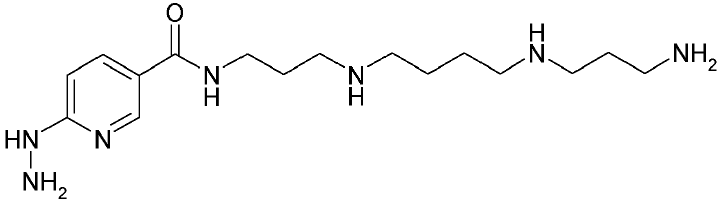

0,74 g de l'ester *tert*-butylique de l'acide (3-*tert*-butoxycarbonylamino-propyl)-[4-(*tert-*butoxycarbonyl-{3-[(6-hydrazino-pyridine-3-carbonyl)-amino]-propyl}-amino)-butyl]-carbamique sont dissous dans 20 mL de solution 4 M d'acide chlorhydrique dans le dioxane. Le milieu réactionnel obtenu est laissé sous agitation 7 heures à température ambiante. Le précipité résultant est filtré sous vide, rincé à l'éther éthylique et séché sous vide pour donner 0,45 g de solide de couleur crème. (Rdt : 87 %).
Pf : 282°C.
CCM SiO₂ : CH₂Cl₂/methanol/NH₄OH (40/40/20). Rf: 0,16.
C₁₆H₃₁N₇O : 337.472, sel C₁₆H₃₁N₇O, 4 HCl : 483.332 - Masse: 338.2 (M+H) ESI+ 400°C.
Analyse HPLC sur colonne Waters Atlantis HILIC, 5 µ, 4,6x150 mm
Élution : Acétonitrile/eau/formiate d'ammonium 700/300/0,63 g pH 2,5, débit 1 mL/minute,
λ : 220 nm. Temps rétention : 11.35 min.
1H-RMN (400MHz, DMSO-d6) δ = 8.64 (1H, s, H-2 Ar), 8.16(1H, d, j = 8.8 Hz, H-4 Ar), 6.94 (1H, d, j = 8.8 Hz, H-5 Ar), 3.34 (2H, m, CH₂NHCO), 2.93 (8H, m, CH₂NH₂), 1.98 (4H, m, CH2), 1.88 (4H, m, H2C-CH2).

### Exemple 10 : Préparation de l'ester tert-butylique de l'acide [3-(tert-butoxycarbonyl-{4-[tert-butoxycarbonyl-(3-tert-butoxycarbonyl amino-propyl)-amino]-butyl}-amino)-propyl]-{3-[(6-chloro-pyridine-3-carbonyl)-amino]-propyl}-carbamique

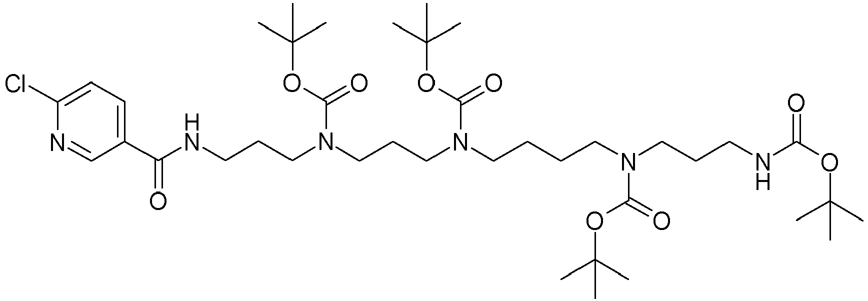

Au mélange de 0,21 g d'acide 6-chloronicotinique et de 0,88 g de (*N*1,*N*4,*N*9,*N*13-tétra-*tert*-butoxycarbonyl)-1,16-diamino-4,9,13-triazahexadécane (Tetrahedron 2000, 56 2449-2460) en solution dans 100 mL d'acétonitrile à température ambiante sous agitation et en présence de 0,22 mL de triéthylamine sont ajoutés en une fois 0,43 g de TBTU. Le mélange est laissé en contact à cette température environ 5 heures. Le milieu réactionnel est hydrolysé avec 100 mL de solution aqueuse d'acide chlorhydrique 0,5M, puis extrait avec de l'acétate d'éthyle (3x50 mL). Après décantation, séchage sur sulfate de sodium anhydre et filtration, le solvant est évaporé sous pression réduite. Le résidu obtenu est purifié par flash chromatographie SiO₂ avec un gradient allant de 100% de CH₂Cl₂ au mélange CH₂Cl₂/Méthanol/NH₄OH (80/18/2) pour obtenir 0,66 g d'huile incolore après évaporation des fractions concernées. (Rdt : 62 %).
CCM SiO₂ : CH₂Cl₂/Méthanol/NH₄OH (90/9/1). Rf : 0,53.

### Exemple 11 : Préparation de l'ester tert-butylique de l'acide [3-(tert-butoxycarbonyl-{4-[tert-butoxycarbonyl-(3-tert-butoxycarbonyl amino-propyl)-amino]-butyl}-amino)-propyl]-{3-[(6-hydrazino-pyridine-3-carbonyl)-amino]-propyl}-carbamique

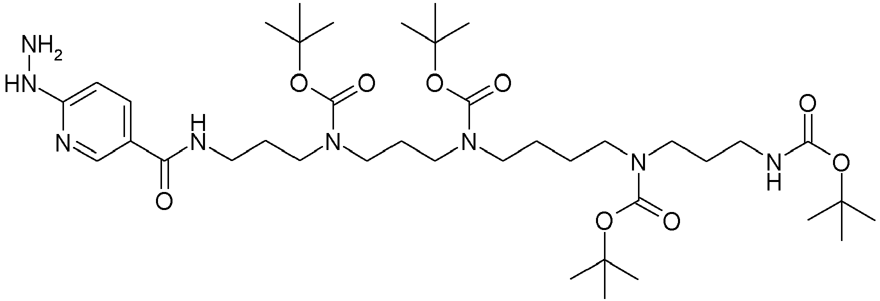

A 0,66 g de l'ester *tert*-butylique de l'acide [3-(*tert*-butoxycarbonyl-14-[*tert-*butoxycarbonyl-(3-*tert*-butoxycarbonyl amino-propyl)-amino]-butyl}-amino)-propyl]-{3-[(6-chloro-pyridine-3-carbonyl)-amino]-propyl}-carbamique sont ajoutés 20 mL d'hydrate d'hydrazine. Le mélange obtenu est porté au reflux pendant environ 5 heures. Après hydrolyse dans 300 mL d'eau, le milieu est extrait à l'acétate d'éthyle (3x100 mL). Les phases organiques sont lavées avec une solution saturée en NaCl puis séchées sur sulfate de sodium anhydre. Après filtration, le solvant est évaporé sous pression réduite pour donner un résidu huileux qui est purifié par flash chromatographie SiO₂ avec un gradient allant de 100 % de CH₂Cl₂ au mélange CH₂Cl₂/Méthanol (80/20) pour obtenir 0,31 g d'huile jaune après évaporation des fractions concernées. (Rdt : 47 %).
CCM SiO₂ : CH₂Cl₂/méthanol/NH₄OH (90/9/1). Rf : 0,28.
C₃₉H₇₀N₈O₉ : 795,041
Analyse HPLC sur colonne Waters X-Brigde C18, 5 µ, 4,6x250 mm
Élution : Acétonitrile/Tampon KH₂PO₄ 6,8 g/L pH 4 (50/50), débit 1 mL/minute,
λ : 220 nm. Temps rétention : 15.07 min.

### Exemple 12 : Préparation du N-(3-{3-[4-(3-amino-propylamino)-butylamino]-propylamino}-propyl)-6-hydrazino-nicotinamide, composé de formule (I) (R1 = R2 = H, a = 3, b = 4, c = 3, d = 2, e = 1)

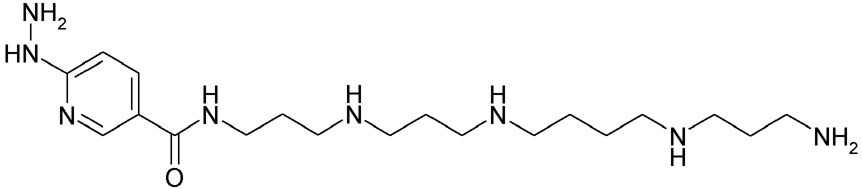

0,31 g de l'ester *tert*-butylique de l'acide ([3-(*tert*-butoxycarbonyl-{4-[*tert-*butoxycarbonyl-(3-*tert*-butoxycarbonyl-amino-propyl)-amino]-butyl}-amino)-propyl]-{3-[(6-hydrazino-pyridine-3-carbonyl)-amino]-propyl}-carbamique sont dissous dans 10 mL de solution 4 M d'acide chlorhydrique dans le dioxane. Le milieu réactionnel obtenu est laissé sous agitation 6 heures à température ambiante. Le précipité résultant est filtré sous vide, rincé à l'éther éthylique et séché sous vide. Une purification par HPLC préparative sur colonne Sunfire C18 OBD de Waters 10 µ, 19x250 mm a été réalisée avec comme phase mobile un gradient allant de HCl 5 mmol à acétonitrile/HCl 5 mmol 50/50 pour obtenir après lyophilisation des fractions concernées 37 mg de penta-chlorhydrate du composé sous forme de poudre grise.
(Rdt : 16 %).
Pf : 307°C.
C₁₉H₃₈N₈O : 394,568, sel C₁₉H₃₈N₈O, 5 HCl : 576.870 - Masse (ESI + 400°C) : 365,2 (M-NH-NH2).
Analyse HPLC sur colonne Waters Atlantis HILIC, 5 µ, 4,6x150 mm
Élution : Acétonitrile/eau/formiate d'ammonium 650/350/0,63 g pH 2, débit 1 mL/minute,
λ : 220 nm. Temps rétention : 7,33min.
¹H-RMN (400MHz, DMSO-d6) δ = 8.63 (1H, s, H-2 Ar), 8.17 (1H, d, j = 8.8 Hz, H-4 Ar), 6.95 (1H, d, j = 8.8 Hz, H-5 Ar), 3.35 (2H, m, CH₂NHCO), 2.98 (12H, m, CH₂NH₂), 1.97 (6H, m, CH2), 1.88 (4H, m, H2C-CH2).

### Exemple 13 : Préparation de l'ester benzylique de l'acide (3-benzyloxycarbonyl-amino-propyl)-(4-{benzyloxycarbonyl-[3-({6-[N'-(2, 2, 2-trifluoro-acétyl)-hydrazino]-pyridine-3-carbonyl}-amino)-propyl]-amino}-butyl)-carbamique

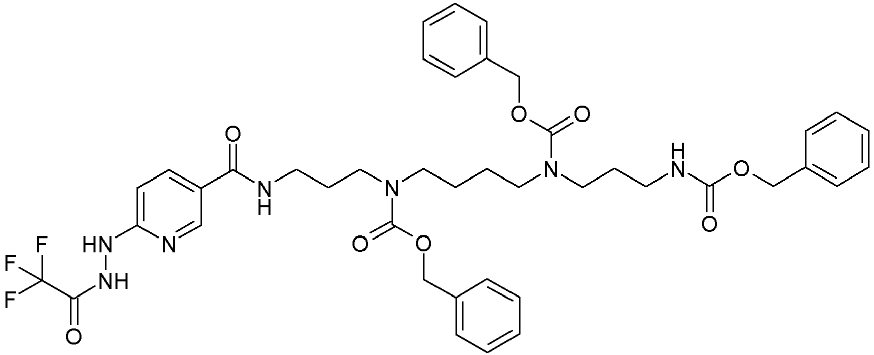

Au mélange de 0,49 g d'acide 6-(2-(2, 2, 2-trifluoroacétyl)hydrazinyl)nicotinique et de 1,2 g de tri-Z-spermine (Tetrahedron Letters 1998, 39, 439-442) en solution dans 12 mL de DMF à température ambiante sous agitation et en présence de 0,33 mL de triéthylamine sont ajoutés en une fois 0,63 g de TBTU. Le mélange est laissé en contact à cette température environ 5 heures. Le milieu réactionnel est hydrolysé avec 100 mL de solution aqueuse d'acide chlorhydrique 0,5 M, puis extrait avec de l'acétate d'éthyle (3x50 mL). Après décantation, séchage sur sulfate de sodium anhydre et filtration, le solvant est évaporé sous pression réduite. Le résidu obtenu est purifié par flash chromatographie SiO₂ avec un gradient allant de 100 % heptane à 100 % acétate d'éthyle pour obtenir 1,23 g de solide jaune. Le chlorhydrate est précipité dans le dichlorométhane par addition de 1 équivalent d'une solution 4 M d'acide chlorhydrique dans le dioxane pour obtenir 0,93 g de solide jaune pâle. (Rdt : 56 %).
CCM SiO₂ : CH₂Cl₂/Méthanol/NH₄OH (90/9/1). Rf : 0,2.
Analyse HPLC sur colonne Waters X-Brigde C18, 5 µ, 4,6x250 mm
Élution : Acétonitrile/Tampon KH₂PO₄ 6,8 g/L pH 4 (60/40), débit 1 mL/minute.
λ : 220 nm. Temps rétention : 7.69 min.

### Exemple 14 : Préparation du N-{3-[4-(3-amino-propylamino)-butylamino]-propyl}-6-[N'-(2, 2, 2-trifluoro-acétyl)-hydrazino]-nicotinamide, composé de formule (I) (R1 = CF₃CO, R2 = H, a = 3, b = 4, c = 3, d = e = 1)

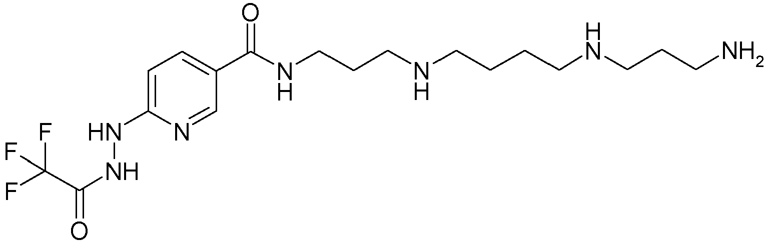

0,52 g de l'ester benzylique de l'acide (3-benzyloxycarbonylamino-propyl)-(4-{benzyloxycarbonyl-[3-({6-[N'-(2, 2, 2-trifluoro-acétyl)-hydrazino]-pyridine-3-carbonyl}-amino)-propyl]-amino}-butyl)-carbamique en solution dans 30 mL de méthanol en présence de Pd/10 % est agité violemment sous atmosphère d'hydrogène à température ambiante pendant 5 heures. Le catalyseur est filtré sous vide, rincé au méthanol. Le filtrat est évaporé sous pression réduite sans chauffer pour obtenir un résidu huileux. Le tétra-chlorhydrate est précipité par addition de 4 équivalents d'une solution 4 M d'acide chlorhydrique dans le dioxane à une solution du résidu obtenu dans le dichlorométhane. Le précipité beige est filtré, rincé à l'éther éthylique, puis séché sous vide pour donner 162 mg de sel. (Rdt : 40 %).
Pf : 237,5°C
C₁₈H₃₀F₃N₇O₂ : 433,481, sel C₁₈H₃₀F₃N₇O₂, 4 HCl : 579,341 - Masse (APCI + 500°C) : 434,2 (M+H)
Analyse HPLC sur colonne Waters Atlantis HILIC, 5 µ, 4,6x150 mm
Élution : Acétonitrile/eau/formiate d'ammonium 700/300/0,63 g pH 2,5, débit 1 mL/minute,
λ : 220 nm. Temps rétention : 5.62 min.
¹H-RMN (400MHz, DMSO-d6) δ = 8.58 (1H, s, H-2 Ar), 7.98 (1H, d, j = 8.8 Hz, H-4 Ar), 6.74 (1H, d, j = 8.8 Hz, H-5 Ar), 3.34 (2H, m, CH₂NHCO), 2.95 (8H, m, CH₂NH₂), 1.95 (4H, m, CH2), 1.68 (4H, m, H2C-CH2).

### Exemple 15 : Préparation de l'ester benzylique de l'acide (3-benzyloxycarbonylamino-propyl)-[4-(benzyloxycarbonyl-{3-[(6-chloro-pyridine-3-carbonyl)-amino]-propyl}-amino)-butyl]-carbamique

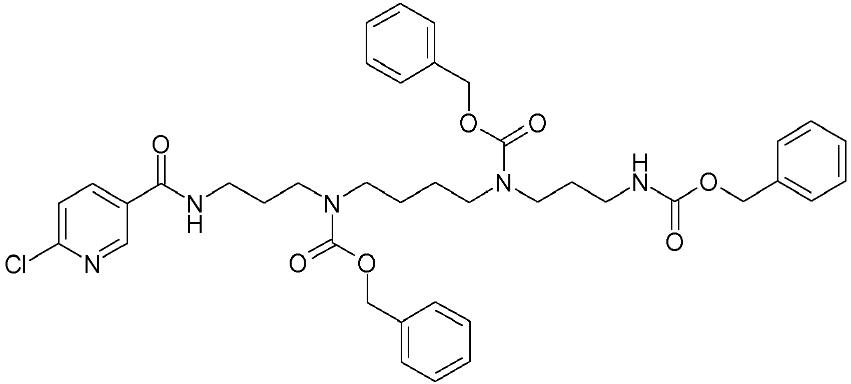

Au mélange de 0,98 g d'acide 6-chloronicotinique et de 3,74 g de tri-Z-spermine (Tetrahedron Letters 1998, 39, 439-442) en solution dans 100 mL d'acétonitrile à température ambiante sous agitation et en présence de 1,05 mL de triéthylamine sont ajoutés en une fois 2 g de TBTU. Le mélange est laissé en contact à cette température environ 90 minutes. Le milieu réactionnel est hydrolysé avec 100 mL de solution aqueuse d'acide chlorhydrique 0,5 M puis extrait avec de l'acétate d'éthyle (3x50 mL). Après décantation, séchage sur sulfate de sodium anhydre et filtration, le solvant est évaporé sous pression réduite. Le résidu obtenu est purifié par flash chromatographie SiO₂ avec un gradient allant de 100 % heptane à 100 % acétate d'éthyle pour obtenir 1,6 g d'huile jaune. (Rdt : 35 %).
CCM SiO₂ : CH₂Cl₂/Méthanol/NH₄OH (90/9/1). Rf : 0,45.
C₄₀H₄₆ClN₅O₇ : 744,295

### Exemple 16 : Préparation de l'ester benzylique de l'acide (3-benzyloxycarbonylamino-propyl)-[4-(benzyloxycarbonyl-{3-[(6-hydrazino-pyridine-3-carbonyl)-amino]-propyl}-amino)-butyl]-carbamique

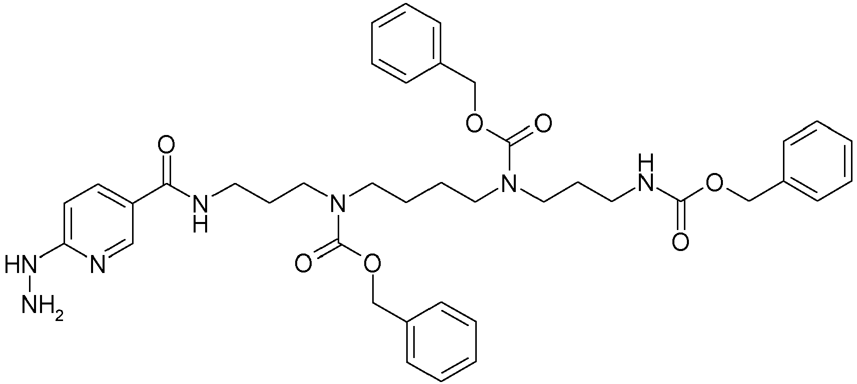

A 1,6 g de l'ester benzylique de l'acide (3-benzyloxycarbonylamino-propyl)-[4-(benzyloxycarbonyl-{3-[(6-chloro-pyridine-3-carbonyl)-amino]-propyl}-amino)-butyl]-carbamique sont ajoutés 20 mL d'hydrate d'hydrazine. Le mélange obtenu est porté au reflux pendant environ 2 heures. Après hydrolyse dans 300 mL d'eau, le milieu est extrait à l'acétate d'éthyle (3x100 mL). Les phases organiques sont lavées avec une solution saturée en NaCl puis séchées sur sulfate de sodium anhydre. Après filtration, le solvant est évaporé sous pression réduite pour donner 1,8 g de résidu huileux. (Rdt : quantitatif).
CCM SiO₂ : CH₂Cl₂/méthanol/NH₄OH (90/9/1). Rf : 0,40.
C₄₀H₄₉N₇O₇ : 739,879 - Masse (ESI + 400°C) : 740,5 (M+H)

### Exemple 17 : Préparation de l'ester tert-butylique de l'acide N'-{5-[3-(benzyloxycarbonyl-{4-[benzyloxycarbonyl-(3-benzyloxycarbonylamino-propyl)-amino]-butyl}-amino)-propylcarbamoyl]-pyridin-2-yl}-hydrazinecarboxylique

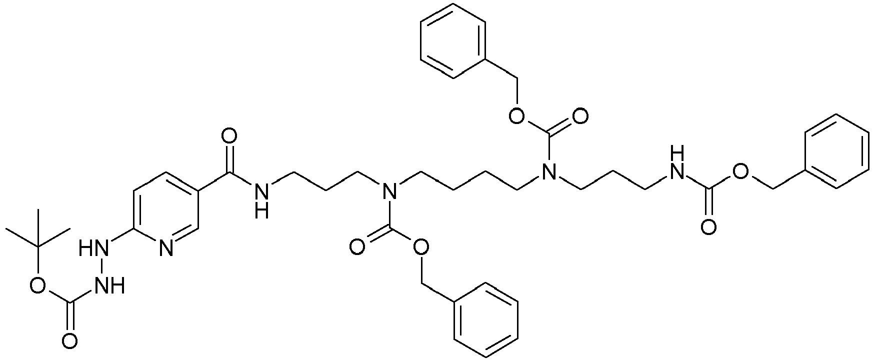

A 1,6 g de l'ester benzylique de l'acide (3-benzyloxycarbonylamino-propyl)-[4-(benzyloxycarbonyl-{3-[(6-hydrazino-pyridine-3-carbonyl)-amino]-propyl}-amino)-butyl]-carbamique en solution dans 30 mL de THF en présence de 2 mL de triéthylamine est ajoutée à température ambiante et goutte à goutte une solution de 2 g de di-*tert*-butyl carbonate dans 10 mL de THF. En fin d'addition, le milieu réactionnel est laissé 2 heures sous agitation. Après hydrolyse dans 300 mL d'eau, le milieu est extrait à l'acétate d'éthyle (3x100 mL). Les phases organiques sont lavées avec une solution saturée en NaCl puis séchées sur sulfate de sodium anhydre. Après filtration, le solvant est évaporé sous pression réduite pour donner résidu huileux qui est purifié par flash chromatographie SiO₂ avec un gradient allant de 100 % heptane à 100 % acétate d'éthyle pour obtenir 1,15 g d'huile incolore. (Rdt : 63 %).
C₄₅H₅₇ClN₇O₉: 839,997
Analyse HPLC sur colonne Waters X-Brigde C18, 5 µ, 4,6x250 mm
Élution : Acétonitrile/Tampon KH₂PO₄ 6,8 g/L pH 4 (60/40), débit 1 mL/minute.
λ : 220 nm. Temps rétention : 9.33 min.

### Exemple 18 : Préparation de l'ester tert-butylique de l'acide N'-(5-{3-[4-(3-amino-propylamino)-butylamino]-propylcarbamoyl}-pyridin-2-yl)-hydrazinecarboxylique, composé de formule (I) (R1 = BOC, R2 = H, a = 3, b = 4, c = 3, d = e = 1).

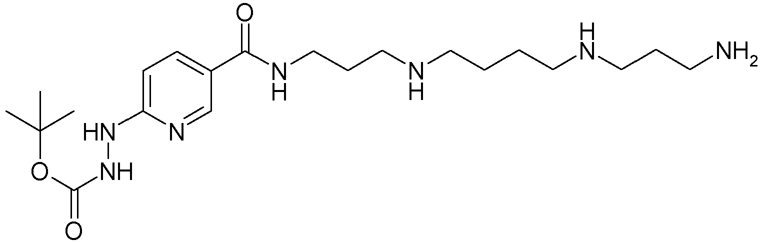

0,2 g de l'ester benzylique de l'acide (3-benzyloxycarbonylamino-propyl)-[4-(benzyloxycarbonyl-{3-[(6-hydrazino-pyridine-3-carbonyl)-amino]-propyl}-amino)-butyl]-carbamique en solution dans 10 mL de méthanol en présence de Pd/10 % est agité violemment sous atmosphère d'hydrogène à température ambiante pendant 2 heures. Le catalyseur est filtré sous vide, rincé au méthanol. Le filtrat est évaporé sous pression réduite sans chauffer pour obtenir 110 mg de solide. (Rdt : quantitatif).
C₂₁H₃₉N₇O3₂ : 437,590 - Masse (ESI + 400°C) : 438,3 (M+H).
Analyse HPLC sur colonne Waters X-bridge C18, 5µ, 4,6x250mm
Élution : Acétonitrile/tampon KH₂PO₄ 6,4 g/l 90/10 pH 4, débit 1 mL/minute λ : 220 nm. Temps rétention : 7,56 min
¹H-RMN (400MHz, DMSO-d6) δ = 8.64 (1H, s, H-2 Ar), 8.16(1H, d, j = 8.8 Hz, H-4 Ar), 6.94 (1H, d, j = 8.8 Hz, H-5 Ar), 3.34 (2H, m, CH₂NHCO), 2.93 (8H, m, CH₂NH₂), 1.98 (4H, m, CH2), 1.88 (4H, m, H2C-CH2), 1.42 (9H, *tert*-butyl).

### Exemple 19 : Préparation de l'ester tert-butylique de l'acide [4-(tert-butoxycarbonyl-{3-[(6-chloro-pyridine-3-carbonyl)-méthyl-amino]-propyl}-amino)-butyl]-carbamique

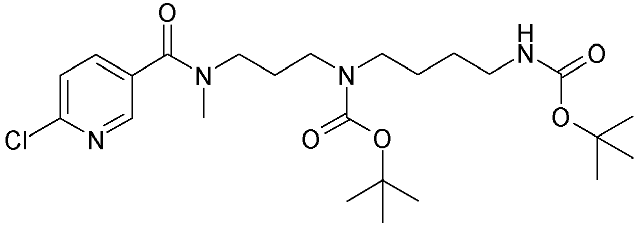

Synthèse réalisée selon le protocole de synthèse de l'ester benzylique de l'acide [4-(benzyloxycarbonyl-{3-[(6-chloro-pyridine-3-carbonyl)-amino]-propyl}-amino)-butyl]-carbamique mais en utilisant l'amine suivante : ester tert-butylique de l'acide {4-[*tert-*butoxycarbonyl-(3-méthylamino-propyl)-amino]-butyl}-carbamique (J. Med. Chem. 1999, 42, 277-290).

### Exemple 20 : Préparation de l'ester tert-butylique de l'acide [4-(tert-butoxycarbonyl-{3-[(6-hydrazino-pyridine-3-carbonyl)-méthyl-amino]-propyl}-amino)-butyl]-carbamique

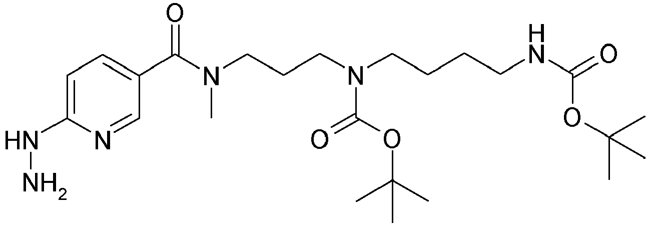

Réaction avec l'hydrazine suivant les protocoles décrits précédemment.

### Exemple 21 : Préparation du N-[3-(4-Amino-butylamino)-propyl]-6-hydrazino-N-methyl-nicotinamide, composé de formule (I) (R1 = H, R2 = CH₃, b = 3, c = 4, d = 0, e = 1).

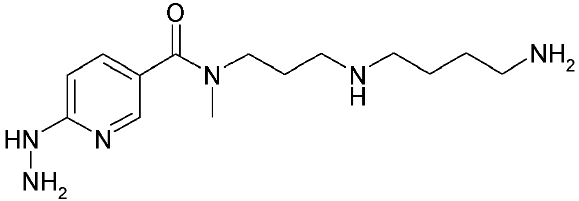

Déprotection avec une solution 4 M d'acide chlorhydrique dans le dioxane suivant les protocoles décrits précédemment.

### Exemple 22 : Dérivé de type AADT synthétisé

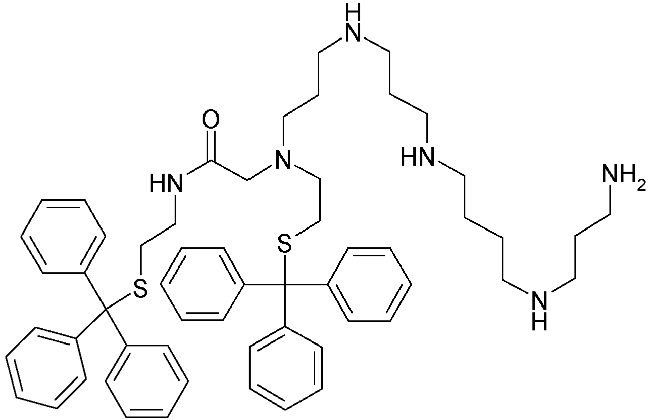

A un mélange de 0,5 g (1 éq., 0,66 mmole) de N-[[[2-[(Triphénylméthyl) thio]éthyl]amino]carbonyl]-méthyl]-*N*-(3'-chloropropyl)-*S*-(triphénylméthyl)-2-aminoéthanethiol (décrit dans J. Med. Chem. 1997, 40, 1835-1844) et de 1,34 g de spermine (10 éq., 6,6 mmoles) en solution dans 100 mL de mélange 1/1 de dichlorométhane et de méthanol sont additionnés 50 mg de bromure de tétrabutylammonium. Ce mélange obtenu est évaporé sous pression réduite pour obtenir un résidu huileux qui est chauffé à 110°C pendant 9 heures. Après refroidissement, 50 mL de soude 1 N sont ajoutés et une extraction au dichlorométhane (4x100 mL) est effectuée. Les phases organiques d'extraction sont rassemblées, séchées sur sulfate de sodium anhydre, filtrées et évaporées sous pression réduite pour obtenir un résidu huileux qui est purifié par flash chromatographie sur gel de silice avec un gradient allant de 100% de dichlorométhane à dichlorométhane/méthanol/ammoniaque à 37 % (70/20/10) pour obtenir 0,27 g (rdt : 37 %) sous forme d'une huile orange. Une purification complémentaire par HPLC préparative est réalisée sur colonne C18-Xbridge de Waters 30x250mm, 10 m, débit 40ml/min., I : 220 nm, phase mobile : gradient 100 % acétonitrile à acétonitrile/solution aqueuse HCl 5mmolaire (50/50).

Les fractions concernées par le produit sont évaporées pour éliminer l'acétonitrile puis lyophilisées pour obtenir le produit sous forme de solide crème et sous sa forme chlorhydrate. Rdt : 12 %. CCM : CH₂Cl₂/méthanol/ammoniaque 4/4/2 : Rf : 0,5

### Exemple 23 : Complexation du composé de l'exemple 22 avec le Technétium 99m

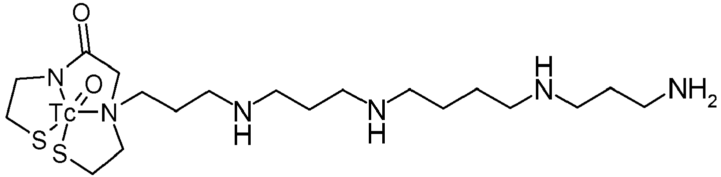

Dans un ballon de 100 ml équipé d'une agitation magnétique, on ajoute au composé de l'exemple 22 (5 mg) de l'anisole (0,2 ml ; 1,8 mmol) puis de l'acide trifluoroacétique 99 % (10 ml ; 134 mmol) qui donne une coloration jaune à la solution. Le milieu réactionnel est ensuite agité 5 min à 5°C. Le mélange est ensuite titré, goutte à goutte, avec du triéthylsilane (Et₃Si) (0,07 ml ; 0,43 mmol) jusqu'à disparition de la couleur jaune. La solution est évaporée sous pression réduite à température ambiante. Le composé obtenu (1 mg) est dissout dans du NaCl 0,9 % (1 ml) puis ajouté, dans un flacon sous vide, à un mélange de fluorure d'étain (SnF₂) (80 µg ; 0,51 µmol) et d'acide ascorbique (0,5 mg; 2,8 µmol). On laisse ensuite incuber pendant 3 minutes à température ambiante, avant de rajouter le pertechnétate de sodium (500 µL; 185 MBq). Celui-ci, réduit extemporanément du degré d'oxydation +VII au degré +III, est alors complexé sous forme de pentadentate. Après incubation de 30 minutes, le volume est ajusté à 4 ml avec du NaCl 0,9 %.

### Exemple 24 : Etudes in vivo avec le complexe de l'exemple 23

Fixation tumorale : Des cellules de cancer du sein MX1 (5.10⁶ cellules) ont été injectées en sous-cutané au niveau du flanc de souris femelles Swiss nude. 22 jours post-greffe, la sonde radiomarquée (15 MBq5) a été injectée à ces souris. Des scintigraphies du corps entier ont ensuite été réalisées 30 min, 1 h et 5 h post-injection. Pour cela, les souris, anesthésiées par voie gazeuse avec de l'isoflurane, ont été imagées en décubitus ventral sur une gamma-caméra (γ Imager, BIOSPACE Mesures) avec le logiciel γ Acquisition (BIOSPACE Mesures) avec les paramètres suivants :
- Fenêtre spectrale : 124-160 KeV
- Temps d'acquisition : 5 min
- Matrice 256x256

Pour l'ensemble des images, une région d'intérêt a été tracée autour de la tumeur et une autre de même taille a été définie sur le muscle d'une patte arrière afin de déterminer le rapport tumeur/muscle.

Les résultats indiquent qu'il n'y a pas de fixation tumorale, le traceur se concentre rapidement dans le foie. Ainsi, ce traceur n'est pas reconnu par le système de transport des polyamines.

### Exemple 25 : Chélation directe du Technétium 99m sur les amines de la spermine après réduction du TcO₄⁻

La spermine (10 mg) est dissoute dans de l'eau ppi (1 ml). 100 µL de la solution obtenue sont ajoutés, dans un flacon sous vide, à du fluorure d'étain (80 µg ; 0,51 µmol). On rajoute ensuite le pertechnétate de sodium (370 MBq). Celui-ci, réduit extemporanément du degré d'oxydation +VII au degré +III, est alors complexé sous forme de monodentate. Puis on ajoute de l'acide ascorbique (0,5 mg ; 2,8 µmol). Après incubation de 15 minutes à température ambiante, le pH est ajusté aux environs de 6,3-6,7.

### Exemple 26 : Etudes in vivo avec le composé de l'exemple 25

Fixation tumorale : Des cellules de mélanome murin B16/F10 (2,5.10⁵ cellules) ont été injectées en sous-cutané au niveau de la patte arrière droite de souris males C57BL/6. 10 jours post-greffe, la sonde radiomarquée a été injectée aux souris. L'imagerie a été réalisée selon le protocole décrit dans l'exemple 24 ci-dessus.

Les résultats indiquent qu'il n'y a pas de fixation tumorale, une partie du traceur est rapidement éliminé par voie urinaire et il y a une forte fixation hépatique. Ainsi, le complexe de l'exemple 25 n'est pas reconnu par le système de transport des polyamines.

### Exemple 27 : Complexation des composés de formule (I) avec le Technétium 99m

Un exemple de marquage est donné avec le N-(3-{3-[4-(3-amino-propylamino)-butylamino]-propylamino}-propyl)-6-hydrazino-nicotinamide, composé de formule (I) (R1 = R2 = H, a = 3, b = 4, c = 3, d = e = 1), permettant de donner le complexe suivant :

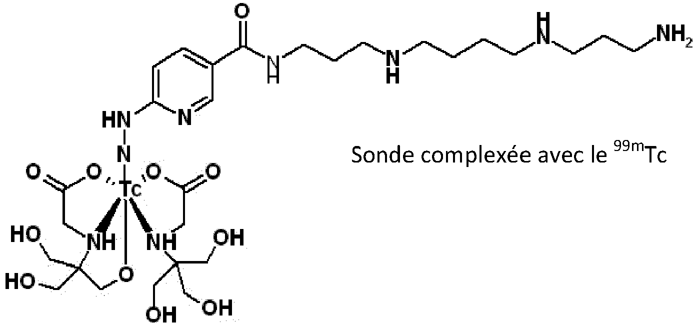

Le composé de formule (I) (1,5 mg) et de la tricine (24 mg ; 89 µmol) sont dissous dans du NaCl 0,9% (1,5 ml) puis 1 ml de la solution obtenue est placée dans un flacon sous vide. Du fluorure d'étain (80 µg ; 0,51 µmol) et de l'acide ascorbique (0,5 mg ; 2,8 µmol) sont ajoutés comme agents réducteurs. On laisse ensuite incuber pendant 3 minutes à température ambiante, avant d'ajouter le pertechnétate de sodium (500 µL ; 370 MBq). Celui-ci, réduit extemporanément du degré d'oxydation +VII au degré +III, est alors complexé sous forme de monodentate. Après incubation de 30 minutes, du PBS 10x est ajouté (0,5ml) puis le volume est ajusté à 5ml avec du NaCl 0,9 %.

### Exemple 28 : Contrôle du marquage

Une radio HPLC en phase inverse a été réalisée sur une colonne XBridge C8 4,6*250mm 5 µ (Waters, USA) avec une phase mobile (débit de 1 ml/min à température ambiante) composée d'eau (55 %), d'acétonitrile (45 %) et de TFA(0,1 %). Aucune étape de purification n'a été réalisée avant injection.

Des radios HPLC en phase inverse réalisées entre 30 min et 5 h post-marquage des composés ont montré que le marquage est stable.

Les résultats obtenus à 30 min post-marquage sont présentés dans le tableau ci-dessous.

| **Composé de formule (II)** | | | | | | **Temps de rétention** | **Pureté radiochimique (%)** |
|---|---|---|---|---|---|---|---|
| **R2** | **a** | **b** | **c** | **d** | **e** | | |
| H⁽¹⁾ | 3 | 4 | 3 | 1 | 1 | 3min20 | 99 |
| H | 4 | / | / | 1 | 0 | 2min57 | 99 |
| H | 3 | 4 | 3 | 2 | 1 | 2min46 | 98 |
| H | / | 3 | 4 | 0 | 1 | 2min52 | 98 |
| H⁽²⁾ | 3 | 4 | 3 | 1 | 1 | 2min33 | 99 |
| **99mTc** | | | | | | 4min41 | / |

| | | | | | | | |
|---|---|---|---|---|---|---|---|
| ⁽¹⁾ Complexe de formule (II) obtenu à partir d'un composé de formule (I) pour lequel R1 = H. ⁽²⁾ Complexe de formule (II) obtenu à partir d'un composé de formule (I) pour lequel R1 = CF₃CO. | | | | | | | |

### Exemple 29 : Etude in vitro - Incorporation dans différents types cellulaires du complexe de formule (II) obtenu à partir d'un composé de formule (I) pour lequel R1 = R2 = H, a = 3, b = 4, c = 3, d = e = 1

Des cellules A549 et B16 ont été mises en culture dans des plaques à 24 puits (2.10⁵ cellules/puits). Après 24h, les cellules ont été incubées pendant 30 minutes avec le complexe de formule (II) à différentes concentrations ou avec 0,2 µM de complexe de formule (II) en présence ou non d'une concentration croissante de spermine utilisée pour évaluer la compétition avec la sonde. Après l'incubation, les plaques ont été placées sur la glace et les cellules ont été rincées avec du NaCl 0,9 % froid additionné de 1 mM de spermidine. Les cellules ont reçu un traitement à la trypsine. Elles ont été mises en suspension dans du PBS. L'activité de chaque puits a été mesurée à l'aide d'un compteur gamma.

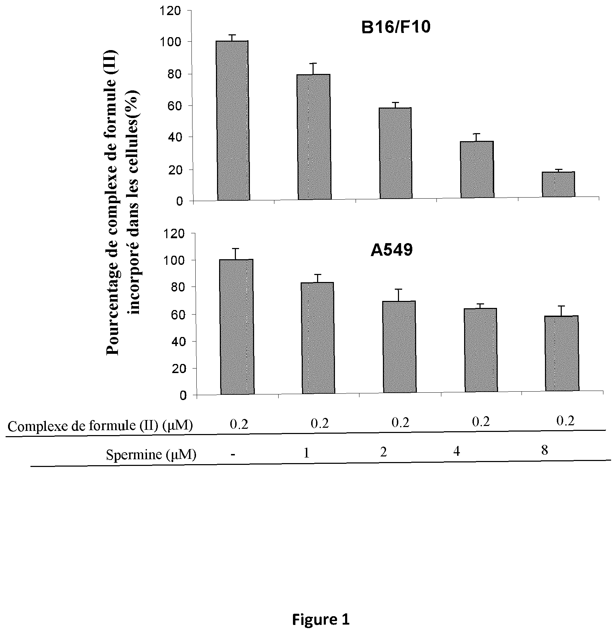

La spermine bloque en partie l'incorporation du complexe de formule (II) dans les cellules B16 et A549 (résultats présentés sur la Figure 1), ce qui indique que la sonde emprunte les transporteurs de polyamines pour entrer dans les cellules.

### Exemple 30 : Etudes in vivo avec le complexe de formule (II) obtenu à partir d'un composé de formule (I) pour lequel R1 = R2 = H, a = 3, b = 4, c = 3, d = e = 1

1) Fixation tumorale : Des cellules de mélanome murin B16/F10 (2,5.10⁵ cellules) ont été injectées en sous cutané au niveau de la patte arrière droite de souris mâles C57BL/6. 10 jours post-greffe, le complexe de formule (II) (14,8MBq) a été injecté à 18 souris, 400 µg de spermine ayant été préalablement injectés à 8 de ces souris. Des scintigraphies du corps entier ont ensuite été réalisées 30 min et 2 h post-injection. Pour cela, les souris, anesthésiées par voie gazeuse avec de l'isoflurane, ont été imagées en décubitus ventral sur une gamma caméra (γ Imager, BIOSPACE Mesures) avec le logiciel IRIS (Ariès Nucléaire, France) avec les paramètres suivants :
- Fenêtre spectrale : 121 - 164 KeV
- Temps d'acquisition : 3 min
- Matrice : 256*256

Pour l'ensemble des images, une région d'intérêt a été tracée autour de la tumeur et une autre de même taille a été définie sur le muscle de la patte controlatéral afin de déterminer le rapport tumeur sur muscle. Les résultats obtenus sont présentés dans le tableau qui suit.

| **Temps post-injection du complexe de formule (II)** | **30 min** | | **2 h** | |
|---|---|---|---|---|
| | Sans spermine | Avec spermine | Sans spermine | Avec spermine |
| Rapport tumeur/muscle (moyenne ± écart-type) | 3,97 ± 1,01* | 2,31 ± 0,50 | 5,26 ± 1,40* | 2,93 ± 1,12 |
| Inhibition (%) | 45,0 | | 47,7 | |
| *Différence significative (P<0.001) entre les lots avec ou sans compétiteur | | | | |

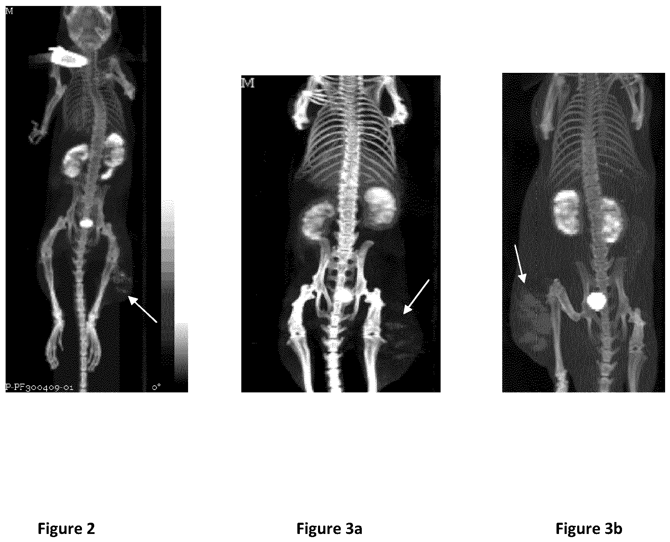

Des souris ont également été imagées en TEMP (nanoSPECT/CT, Bioscan, USA), une image étant présentée sur la Figure 2.

La compétition mise en évidence dans cette étude entre la spermine et le complexe de formule (II) indique que la sonde utilise le système de transport des polyamines. Cette sonde est donc un biomarqueur de l'incorporation de spermine par les cellules. 2) Stabilité plasmatique : 14,8MBq de complexe de formule (II) ont été injectés par voie intraveineuse à des souris mâles C57BL/6 saines. 30 minutes, 1 h, 2 h, 3 h et 5 h post-injection, des prélèvements de sang sur héparine ont été réalisés au sinus rétro-orbitaire. Ces échantillons ont ensuite été centrifugés 5 minutes afin de récupérer au moins 100 µL de sérum. 50 µL ont permis la réalisation d'une radio HPLC d'exclusion-diffusion sur une colonne TSK SW2000 + pré-colonne 7,8*300mm 5 µ (Tosoh bioscience) avec une phase mobile (débit de 1 ml/min à température ambiante) composée de NaCl 0,9 % (100 %). De l'albumine de macaque marqué avec du ^{99m}Tc a été utilisée pour le contrôle du temps de rétention de l'albumine.

Les 50 µL de sérum restants ont permis de réaliser une radio HPLC en phase inverse, après dénaturation des protéines avec de l'acétonitrile, sur une colonne XBridge C8 4,6*250 mm 5 µ (Waters, USA) avec une phase mobile (débit de 1 ml/min à température ambiante) composée d'eau (55 %), d'acétonitrile (45 %) et de TFA (0,1 %). Une grande partie du complexe de formule (II) injecté est éliminé rapidement (38 % à 30 minutes). Une partie du Technétium s'est transchelaté sur l'albumine plasmatique dès 30 minutes après l'injection par voie intraveineuse. La radio HPLC en phase inverse indique une bonne stabilité du complexe. 3) Biodistribution chez la souris saine : Cette étude a été réalisée sur 32 souris C57BL/6 (16 mâles et 16 femelles) âgées de 6-7 semaines. 2,96 MBq de complexe de formule (II) ont été injectés par voie intraveineuse sous un volume de 60 µL. Puis les animaux ont été sacrifiés 30 min, 2 h, 5 h et 24 h post-injection (4 souris/temps) et divers organes ont été prélevés, pesés et leur activité à été déterminée avec un compteur gamma. La quantité de complexe de formule (II) présente dans chaque organe a été calculée en pourcentage de la dose injectée par gramme de tissus (% ID)/g.

Le complexe de formule (II) se distribue de manière quasi homogène dans l'ensemble des organes et sa fixation ne persiste que dans le foie et les reins. La fixation rénale est due au système mégaline/cubiline qui est impliqué dans un processus de récupération et d'épargne de molécules utiles à l'organisme. Plus les molécules sont petites et aminées, plus elles sont récupérées dans l'urine primaire au niveau du tubule proximal où elles sont réabsorbées. Les taux de fixation observés au niveau du foie et des reins suggèrent que chez la souris il n'y a pas de risque de toxicité rénale ni d'effets secondaires dus à la dosimétrie.

### Exemple 31 : Etude in vivo avec d'autres complexes de formule (II)

L'étude a été réalisée selon le protocole décrit dans l'exemple 30, 1) ci-dessus avec d'autres complexes de formule (II).

Les résultats sont présentés dans le tableau suivant :

| **Complexe de formule (II)** | | | | | | **Rapport Tumeur sur Muscle** | Observations |
|---|---|---|---|---|---|---|---|
| **R2** | **a** | **b** | **c** | **d** | **e** | | |
| H⁽¹⁾ | 3 | 4 | 3 | 1 | 1 | 5,3 ± 0.8 | Fixation tumorale |
| H | 4 | / | / | 1 | 0 | 2,6 ± 1,5 | Fixation tumorale |
| H | 3 | 4 | 3 | 2 | 1 | 3,2 ± 0,9 | Fixation tumorale |
| H | / | 3 | 4 | 0 | 1 | 2,1 ± 0,4 | Fixation tumorale |
| H⁽²⁾ | 3 | 4 | 3 | 1 | 1 | 4,6 ± 0,9 | Fixation tumorale |

| | | | | | | | |
|---|---|---|---|---|---|---|---|
| ⁽¹⁾ Complexe de formule (II) obtenu à partir d'un composé de formule (I) pour lequel R1 = H. ⁽²⁾ Complexe de formule (II) obtenu à partir d'un composé de formule (I) pour lequel R1 = CF₃CO. | | | | | | | |

Des souris ont également été imagées en TEMP (nanoSPECT/CT, Bioscan, USA), deux images étant présentées sur les Figures 3a et 3b obtenues avec un composé de formule (II) pour lequel R2 = H, a = 3, b = 4, c = 3, d = e = 1, ce composé de formule (II) ayant été obtenu à partir d'un composé de formule (I) pour lequel R1 = H ou CF₃CO respectivement.

On peut ainsi constater que l'ensemble des sondes se fixe sur la tumeur. Cependant, dans le cas des composés des exemples 3 et 6 (comprenant moins de trois atomes basiques dans le motif polyamine), le rapport tumeur sur muscle est moindre qu'avec les autres composés ce qui permet un moins bon diagnostic de la tumeur.

### Abréviations utilisées dans la partie expérimentale :

- APCI: Ionisation chimique à pression atmosphérique
- BOC: *tert*-Butyloxycarbonyle
- CCM: Chromatographie sur Couche Mince
- DMF: Diméthylformamide
- DMSO: Diméthylsulfoxyde
- ESI: Ionisation par électrospray
- HPLC: Chromatographie liquide haute performance
- PBS: Tampon phosphate salin
- Pf: Point de fusion
- ppi: Pour préparation injectable
- Rdt: Rendement
- Rf: Rapport frontal
- RMN: Résonance magnétique nucléaire
- TEMP: Tomographie d'émission monophotonique
- TFA: Acide trifluoroacétique
- THF: Tétrahydrofurane
- Z: Benzyloxycarbonyle

## Revendications

1. Composé de formule (I) suivante : 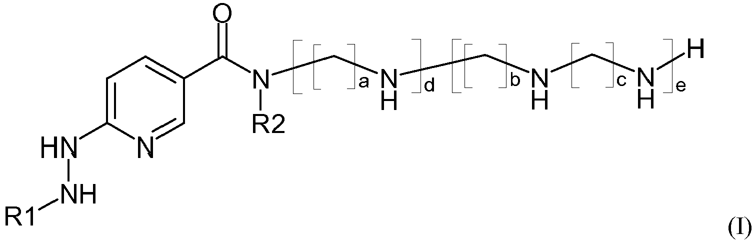 dans laquelle :
- R1 représente un atome d'hydrogène ou un groupement N-protecteur choisi parmi le formyle, l'acétyle, le trifluoroacétyle, le benzoyle, le pivaloyle, le phénylsulfonyle, le benzyle (Bn), le t-butyloxycarbonyle (BOC), le benzyloxycarbonyle (Z), le p-méthoxybenzyloxycarbonyle, le p-nitrobenzyl-oxycarbonyle, le trichloroéthoxycarbonyle (TROC), l'allyloxycarbonyle (Alloc), le 9-fluorénylméthyloxycarbonyle (Fmoc), le trifluoro-acétyle, et les carbamates de benzyle,
- R2 représente un atome d'hydrogène, un groupement C₁₋₆alkyle éventuellement fluoré ou perfluoré, ou un groupement amino-C₁₋₆alkyl,
- a, b et c représentent, indépendamment les uns des autres, un nombre entier de 2 à 5, et
- d et e représentent, indépendamment l'un de l'autre, 1 ou 2,
ou un sel pharmaceutiquement acceptable de celui-ci.

2. Composé selon la revendication 1, **caractérisé en ce que** R2 représente un atome d'hydrogène ou un groupement C₁₋₆alkyle tel que méthyle.

3. Composé selon l'une quelconque des revendications 1 et 2, **caractérisé en ce que** a, b et c représentent, indépendamment les uns des autres, 3 ou 4.

4. Composé selon l'une quelconque des revendications 1 à 3, **caractérisé en ce que** e représente 1 et d représente 1 ou 2.

5. Composé selon l'une quelconque des revendications 1 à 4, caractérisé en ce R1 représente un atome d'hydrogène ou un groupement N-protecteur choisi parmi les groupes *tert*-butyloxycarbonyle (*t*BuOCO) et trifluoroacétyle.

6. Composé selon l'une quelconque des revendications 1 à 5, **caractérisé en ce qu'**il s'agit d'un composé de formule (I) pour lequel :
- R1 = R2 = H, a = 3, b = 4, c = 3, d = 1 et e = 1,
- R1 = R2 = H, a = 3, b = 4, c = 3, d = 2 et e = 1,
- R1 = CF₃CO, R2 = H, a = 3, b = 4, c = 3, d = 1 et e = 1, ou
- R1 = *t*BuOCO, R2 = H, a = 3, b = 4, c = 3 , d = 1 et e = 1,
ou un sel pharmaceutiquement acceptable de celui-ci.

7. Complexe de formule (II) suivante : 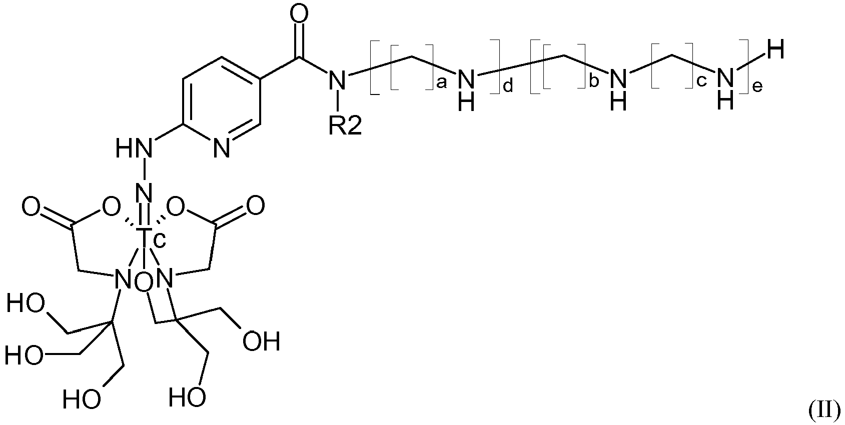 dans laquelle le technétium (Tc) est présent sous forme de son isotope 99m et R2, a, b, c, d et e sont tels que définis dans l'une quelconque des revendications 1 à 4, ou un sel pharmaceutiquement acceptable de celui-ci.

8. Complexe selon la revendication 7, **caractérisé en ce qu'**il s'agit d'un complexe de formule (II) pour lequel :
- R2 = H, a = 3, b = 4, c = 3, d = 1 et e = 1, ou
- R2 = H, a = 3, b = 4, c = 3, d = 2 et e = 1,
ou un sel pharmaceutiquement acceptable de celui-ci.

9. Complexe de formule (II) selon l'une quelconque des revendications 7 et 8 ou d'un sel pharmaceutiquement acceptable de celui-ci pour son utilisation dans le diagnostic *in vivo* d'une tumeur cancéreuse exprimant le système de transport des polyamines, notamment par imagerie médicale par scintigraphie.

10. Composition comprenant au moins un composé de formule (I) selon l'une quelconque des revendications 1 à 6 ou un sel pharmaceutiquement acceptable de celui-ci et au moins un excipient pharmaceutiquement acceptable.

11. Composition diagnostique comprenant au moins un complexe de formule (II) selon l'une quelconque des revendications 7 et 8 ou un sel pharmaceutiquement acceptable de celui-ci et au moins un excipient pharmaceutiquement acceptable.

12. Kit comprenant :
(1) une composition telle que définie à la revendication 10,
(2) de la tricine, et
(3) un agent réducteur tel qu'un mélange de fluorure d'étain et d'acide ascorbique.

13. Procédé de préparation d'une composition diagnostique selon la revendication 11 comprenant le mélange d'une composition selon la revendication 10 avec un sel de pertechnétate-99m, au moins un agent réducteur et de la tricine.

14. Procédé de préparation d'un composé de formule (I) selon l'une quelconque des revendications 1 à 6 ou d'un sel pharmaceutiquement acceptable de celui-ci comprenant les étapes successives suivantes :
(a) réaction de l'acide 6-halogéno-nicotinique avec une polyamine protégée de formule (III) suivante : 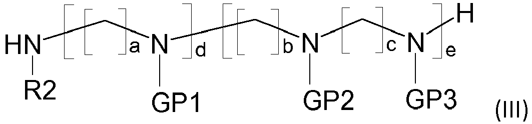 pour laquelle R2, a, b, c, d et e sont tels que définis à la revendication 1 et GP1, GP2 et GP3, identiques ou différents, représentent chacun un groupement N-protecteur,
pour donner un composé de formule (IV) suivante : 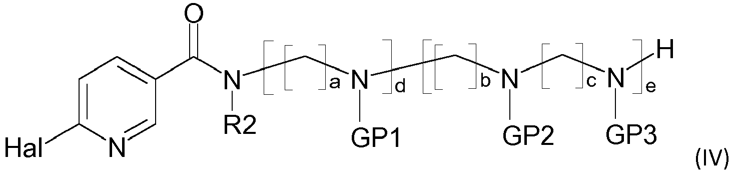 pour laquelle R2, a, b, c, d et e sont tels que définis à la revendication 1, GP1, GP2 et GP3 sont tels que définis précédemment et Hal représente un atome d'halogène,
(b) réaction du composé de formule (IV) obtenu à l'étape (a) précédente avec de l'hydrazine pour donner un composé de formule (V) suivante : 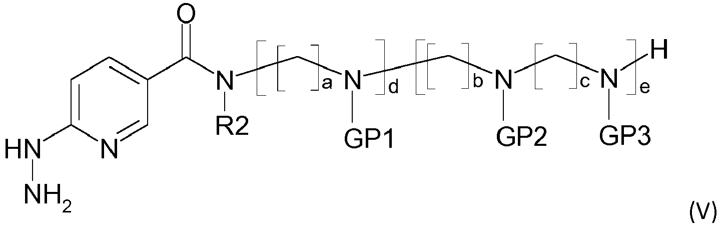 pour laquelle R2, a, b, c, d et e sont tels que définis à la revendication 1 et GP1, GP2 et GP3 sont tels que définis précédemment,
(c) éventuellement protection par un groupement N-protecteur de la fonction hydrazine du composé de formule (V) obtenu à l'étape (b) précédente pour donner un composé de formule (VI) suivante : 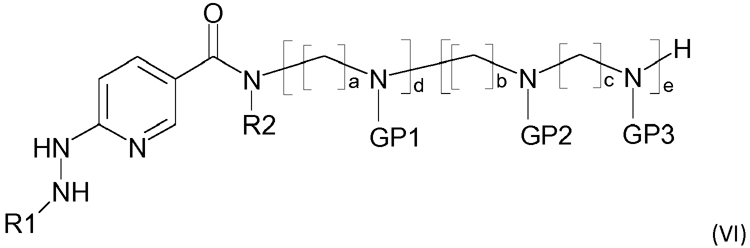 pour laquelle R2, a, b, c, d et e sont tels que définis à la revendication 1, GP1, GP2 et GP3 sont tels que définis précédemment et R1 représente un groupement N-protecteur tel que défini à la revendication 1,
(d) déprotection des fonctions amines protégées par les groupements GP1, GP2 et GP3 dans le composé de formule (V) ou (VI) obtenu à l'étape (b) ou (c) précédente pour donner un composé de formule (I),
(e) éventuellement salification du composé de formule (I) obtenu à l'étape (d) précédente pour obtenir un sel pharmaceutiquement acceptable de celui-ci, et
(f) séparation du composé de formule (I) ou de son sel pharmaceutiquement acceptable obtenu à l'étape précédente du milieu réactionnel.

15. Procédé de préparation d'un composé de formule (I) selon l'une quelconque des revendications 1 à 6 pour lequel R1 ≠ H ou d'un sel pharmaceutiquement acceptable de celui-ci comprenant les étapes successives suivantes :
(i) protection par un groupement N-protecteur de la fonction hydrazine de l'acide 6-hydrazinyl-nicotinique pour donner un composé de formule (VII) suivante : 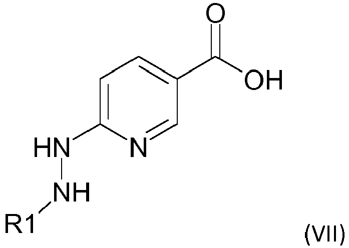 pour laquelle R1 représente un groupement N-protecteur tel que défini à la revendication 1,
(ii) réaction du composé de formule (VII) obtenu à l'étape (i) précédente avec une polyamine protégée de formule (III) telle que définie à la revendication 14 pour donner un composé de formule (VI) tel que défini à la revendication 14,
(iii) déprotection des fonctions amines protégées par les groupements GP1, GP2 et GP3 dans le composé de formule (VI) obtenu à l'étape (ii) précédente pour donner un composé de formule (I) pour lequel R1 ≠ H,
(iv) éventuellement salification du composé de formule (I) obtenu à l'étape (iii) précédente pour obtenir un sel pharmaceutiquement acceptable de celui-ci, et
(v) séparation du composé de formule (I) ou de son sel pharmaceutiquement acceptable obtenu à l'étape précédente du milieu réactionnel.

16. Procédé de préparation d'un complexe de formule (II) selon l'une quelconque des revendications 7 et 8 ou d'un sel pharmaceutiquement acceptable de celui-ci comprenant le mélange d'un composé de formule (I) selon l'une quelconque des revendications 1 à 6 ou d'un sel pharmaceutiquement acceptable de celui-ci avec un sel de pertechnétate-99m, au moins un agent réducteur et de la tricine.

## Patentansprüche

1. Verbindung der folgenden Formel (I): 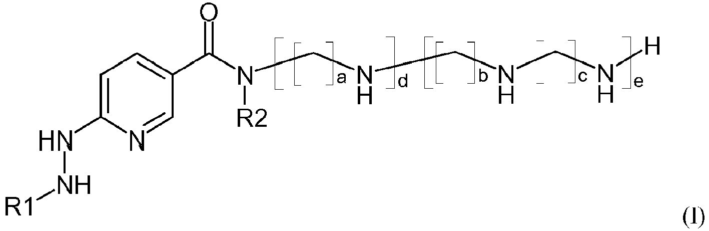 wobei :
- R1 ein Wasserstoffatom oder eine N-Schutzgruppe darstellt, ausgewählt aus dem Formyl, dem Acetyl, dem Trifluoracetyl, dem Benzoyl, dem Pivaloyl, dem Phenylsulfonyl, dem Benzyl (Bn), dem t-Butyloxycarbonyl (BOC), dem Benzyloxycarbonyl (Z), dem p-Methoxybenzyloxycarbonyl, dem p-Nitrobenzyloxycarbonyl, dem Trichlorethoxycarbonyl (TROC), dem Allyloxycarbonyl (Alloc), dem 9-Fluorenylmethyloxycarbonyl (Fmoc), dem Trifluoracetyl und den Benzylcarbamaten,
- R2 ein Wasserstoffatom, eine C₁₋₆alkylgruppe, eventuell fluoriert oder perfluoriert, oder eine Amino-C₁₋₆alkylgruppe darstellt,
- a, b und c unabhängig voneinander eine Ganzzahl von 2 bis 5 darstellen, und
- d und e unabhängig voneinander 1 oder 2 darstellen,
oder ein pharmazeutisch akzeptables Salz davon.

2. Verbindung nach Anspruch 1, **dadurch gekennzeichnet, dass** R2 ein Wasserstoffatom oder eine C₁₋₆alkylgruppe wie Methyl darstellt.

3. Verbindung nach einem der Ansprüche 1 und 2, **dadurch gekennzeichnet, dass** a, b und c unabhängig voneinander 3 oder 4 darstellen.

4. Verbindung nach einem der Ansprüche 1 bis 3, **dadurch gekennzeichnet, dass** e 1 darstellt und d 1 oder 2 darstellt.

5. Verbindung nach einem der Ansprüche 1 bis 4, **dadurch gekennzeichnet, dass** R1 ein Wasserstoffatom oder eine N-Schutzgruppe darstellt, ausgewählt aus dem Gruppen *tert*-Butyloxycarbonyl (*t*BuOCO) und Trifluoracetyl.

6. Verbindung nach einem der Ansprüche 1 bis 5, **dadurch gekennzeichnet, dass** es sich um eine Verbindung der Formel (I) handelt, wobei:
- R1 = R2 = H, a = 3, b = 4, c = 3, d = 1 und e = 1,
- R1 = R2 = H, a = 3, b = 4, c = 3, d = 2 und e = 1,
- R1 = CF₃CO, R2 = H, a = 3, b = 4, c = 3, d = 1 und e = 1, oder
- R1 = *t*BuOCO, R2 = H, a = 3, b = 4, c = 3, d = 1 und e = 1,
oder ein pharmazeutisch akzeptables Salz davon.

7. Komplex der folgenden Formel (II) : 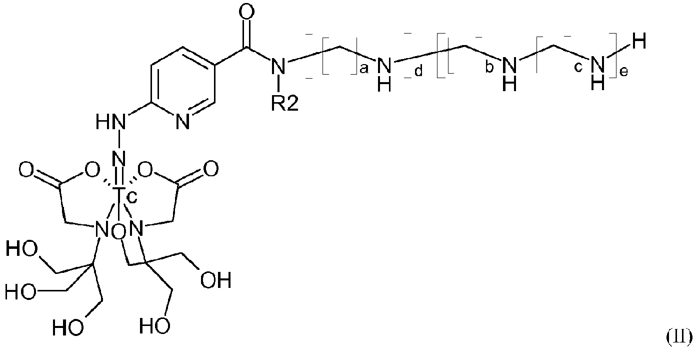 wobei das Technetium (Tc) in Form seines Isotops 99m vorhanden ist und R2, a, b, c, d und e einem der Ansprüche 1 bis 4 entsprechen,
oder ein pharmazeutisch akzeptables Salz davon.

8. Komplex nach Anspruch 7, **dadurch gekennzeichnet, dass** es sich um einen Komplex der Formel (II) handelt, wobei:
- R2 = H, a = 3, b = 4, c = 3, d = 1 und e = 1, oder
- R2 = H, a = 3, b = 4, c = 3, d = 2 und e = 1,
oder ein pharmazeutisch akzeptables Salz davon.

9. Komplex der Formel (II) nach einem der Ansprüche 7 und 8 oder eines pharmazeutisch akzeptablen Salzes davon für seine Verwendung bei der *in vivo-*Diagnose eines kanzerösen Tumors, exprimierend das Transportsystem der Polyamine, insbesondere mittels medizinischer Bildgebung durch Scintigraphie.

10. Zusammensetzung, umfassend mindestens eine Verbindung der Formel (I) nach einem der Ansprüche 1 bis 6 oder ein pharmazeutisch akzeptables Salz davon und mindestens einen pharmazeutisch akzeptablen Hilfsstoff.

11. Diagnostische Zusammensetzung, umfassend mindestens einen Komplex der Formel (II) nach einem der Ansprüche 7 und 8 oder ein pharmazeutisch akzeptables Salz davon und mindestens einen pharmazeutisch akzeptablen Hilfsstoff.

12. Kit, umfassend:
(1) eine Zusammensetzung nach Anspruch 10,
(2) Tricin, und
(3) ein Reduktionsmittel wie ein Gemisch aus Zinnfluorid und Ascorbinsäure.

13. Herstellungsverfahren einer diagnostischen Zusammensetzung nach Anspruch 11, umfassend das Gemisch einer Zusammensetzung nach Anspruch 10 mit einem Salz von Pertechnetat-99m, mindestens einem Reduktionsmittel und Tricin.

14. Herstellungsverfahren einer Verbindung der Formel (I) nach einem der Ansprüche 1 bis 6 oder eines pharmazeutisch akzeptablen Salzes davon, umfassend die folgenden aufeinanderfolgenden Schritte:
(a) Reaktion der 6-Halogennikotinsäure mit einem geschützten Polyamin der folgenden Formel (III) : 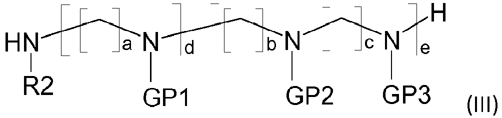 wobei R2, a, b, c, d und e nach Anspruch 1 sind und GP1, GP2 und GP3, identisch oder unterschiedlich, jeweils eine N-Schutzgruppe darstellen,
was eine Verbindung der folgenden Formel (IV) ergibt: 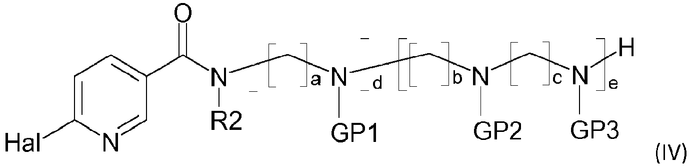 wobei R2, a, b, c, d und e nach Anspruch 1 sind, GP1, GP2 und GP3 wie zuvor definiert sind und Hal ein Halogenatom darstellt,
(b) Reaktion der Verbindung der Formel (IV) aus vorangehendem Schritt (a) mit Hydrazin, was eine Verbindung der folgenden Formel (V) ergibt: 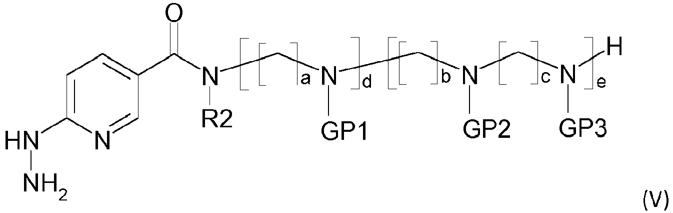 wobei R2, a, b, c, d und e nach Anspruch 1 sind und GP1, GP2 und GP3 wie zuvor definiert sind,
(c) eventuell Schutz durch eine N-Schutzgruppe der Hydrazinfunktion der Verbindung der Formel (V) aus vorangehendem Schritt (b), was eine Verbindung der folgenden Formel (VI) ergibt: 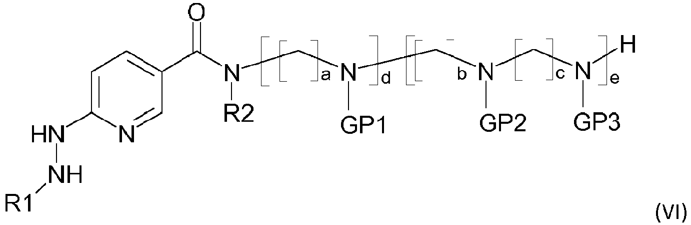 wobei R2, a, b, c, d und e nach Anspruch 1 sind, GP1, GP2 und GP3 wie zuvor definiert sind und R1 eine N-Schutzgruppe nach Anspruch 1 darstellt,
(d) Entschützen der Aminfunktionen, geschützt von den Gruppen GP1, GP2 und GP3 in der Verbindung der Formel (V) oder (VI) aus vorangehendem Schritt (b) oder (c), was eine Verbindung der Formel (I) ergibt,
(e) eventuell Salzbildung der Verbindung der Formel (I) aus vorangehendem Schritt (d), um ein pharmazeutisch akzeptables Salz davon zu erhalten, und
(f) Trennen der Verbindung der Formel (I) oder ihres pharmazeutisch akzeptablen Salzes aus vorangehendem Schritt vom Reaktionsmedium.

15. Herstellungsverfahren einer Verbindung der Formel (I) nach einem der Ansprüche 1 bis 6, wobei R1 ≠ H oder eines pharmazeutisch akzeptablen Salzes davon, umfassend die folgenden aufeinanderfolgenden Schritte:
(i) Schutz durch eine N-Schutzgruppe der Hydrazinfunktion der 6-Hydrazinylnikotinsäure, was eine Verbindung der folgenden Formel (VII) ergibt: 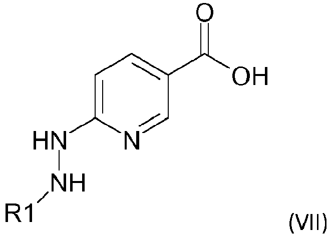 wobei R1 eine N-Schutzgruppe nach Anspruch 1 darstellt,
(ii) Reaktion der Verbindung der Formel (VII) aus vorangehendem Schritt (i) mit einem geschützten Polyamin der Formel (III) nach Anspruch 14, was eine Verbindung der Formel (VI) nach Anspruch 14 ergibt,
(iii) Entschützen der geschützten Aminfunktionen, geschützt von den Gruppen GP1, GP2 und GP3 in der Verbindung der Formel (VI) aus vorangehendem Schritt (ii), was eine Verbindung der Formel (I) ergibt, wobei R1≠ H,
(iv) eventuell Salzbildung der Verbindung der Formel (I) aus vorangehendem Schritt (iii), um ein pharmazeutisch akzeptables Salz davon zu erhalten, und
(v) Trennen der Verbindung der Formel (I) oder ihres pharmazeutisch akzeptablen Salzes aus vorangehendem Schritt vom Reaktionsmedium.

16. Herstellungsverfahren eines Komplexes der Formel (II) nach einem der Ansprüche 7 und 8 oder eines pharmazeutisch akzeptablen Salzes davon, umfassend das Gemisch einer Verbindung der Formel (I) nach einem der Ansprüche 1 bis 6 oder eines pharmazeutisch akzeptablen Salzes davon mit einem Salz von Pertechnetat-99m, mindestens einem Reduktionsmittel und Tricin.

## Claims

1. A compound having the following formula (I): 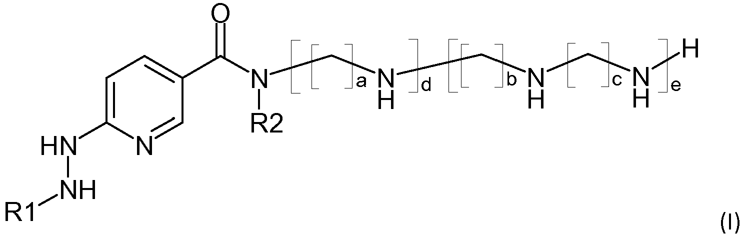 wherein:
- R1 represents a hydrogen atom or an N-protecting group chosen among formyl, acetyl, trifluoroacetyl, benzoyl, pivaloyl, phenylsulfonyl, benzyl (Bn), t-butyloxycarbonyl (BOC), benzyloxycarbonyl (Z), p-methoxybenzyloxycarbonyl, p-nitrobenzyl-oxycarbonyl, trichloroethoxycarbonyl (TROC), allyloxycarbonyl (Alloc), 9-fluorenylmethyloxycarbonyl (Fmoc), trifluoro-acetyl, and benzyl carbamates,
- R2 represents a hydrogen atom, an optionally fluorinated or perfluorinated C₁₋₆alkyl group, or an amino-C₁₋₆alkyl group,
- a, b and c represent, independently of each other, an integer from 2 to 5, and
- d and e represent, independently of one another, 1 or 2,
or a pharmaceutically acceptable salt thereof.

2. The compound according to claim 1, **characterised in that** R2 represents a hydrogen atom or a C₁₋₆alkyl group such as methyl.

3. The compound according to any one of claims 1 and 2, **characterised in that** a, b and c represent, independently from each other, 3 or 4.

4. The compound according to any one of claims 1 to 3, **characterised in that** e represents 1 and d represents 1 or 2.

5. The compound according to any one of claims 1 to 4, **characterized in that** R1 represents a hydrogen atom or an N-protecting group chosen among tert-butoxycarbonyl (tBuOCO) and trifluoroacetyl groups.

6. The compound according to any one of claims 1 to 5, **characterised in that** it consists of a compound having formula (I) wherein:
- R1 = R2 = H, a = 3, b = 4, c = 3, d = 1 and e = 1,
- R1 = R2 = H, a = 3, b = 4, c = 3, d = 2 and e = 1,
- R1 = CF₃CO, R2 = H, a = 3, b = 4, c = 3, d = 1 and e = 1, or
- R1 = *t*BuOCO, R2 = H, a = 3, b = 4, c = 3, d = 1 and e = 1,
or a pharmaceutically acceptable salt thereof.

7. A complex having the following formula (II): 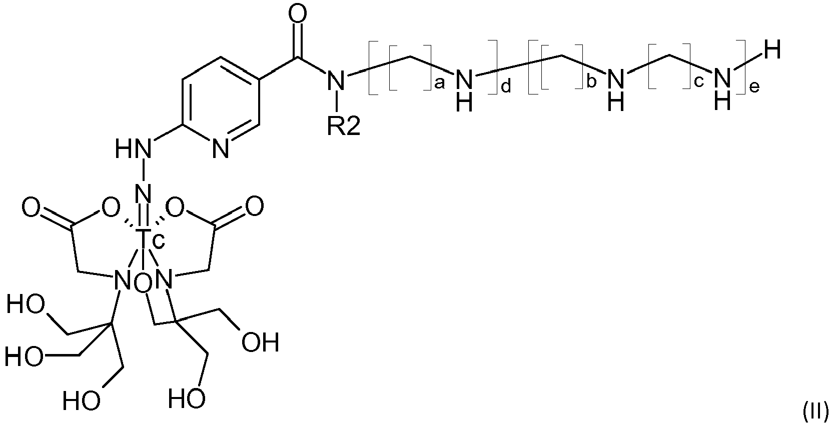 wherein technetium (Tc) is present in the form of the 99m isotope thereof and R2, a, b, c, d and e are as defined in any one of claims 1 to 4,
or a pharmaceutically acceptable salt thereof.

8. The complex according to claim 7, **characterised in that** it consists of a complex having formula (II) wherein:
- R2 = H, a = 3, b = 4, c = 3, d = 1 and e = 1, or
- R2 = H, a = 3, b = 4, c = 3, d = 2 and e = 1,
or a pharmaceutically acceptable salt thereof.

9. A complex having formula (II) according to any of claims 7 and 8 or a pharmaceutically acceptable salt thereof for the use thereof in the *in vivo* diagnosis, of a cancerous tumour expressing the polyamine transport system, particularly by medical imaging, by scintigraphy.

10. A composition comprising at least one compound having formula (I) according to any one of claims 1 to 6 or a pharmaceutically acceptable salt thereof and at least one pharmaceutically acceptable excipient.

11. A diagnostic composition comprising at least one complex having formula (II) according to any one of claims 7 and 8 or a pharmaceutically acceptable salt thereof and at least one pharmaceutically acceptable excipient.

12. A kit comprising:
(1) a composition as defined in claim 10,
(2) tricine, and
(3) a reducing agent such as a mixture of tin fluoride and ascorbic acid.

13. A process for preparing a diagnostic composition according to claim 11 comprising the mixture of a composition according to claim 10 with a pertechnetate-99m salt, at least one reducing agent and tricine.

14. A process for preparing a compound having formula (I) according to any of claims 1 to 6 or a pharmaceutically acceptable salt thereof comprising the following successive steps:
(a) reacting 6-halo-nicotinic acid with a protected polyamine having the following formula (III): 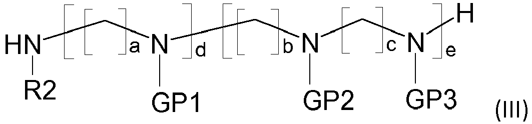 wherein R2, a, b, c, d and e are as defined in claim 1 and GP1, GP2 and GP3, identical or different, each represent an N-protecting group,
to obtain a compound having the following formula (IV): 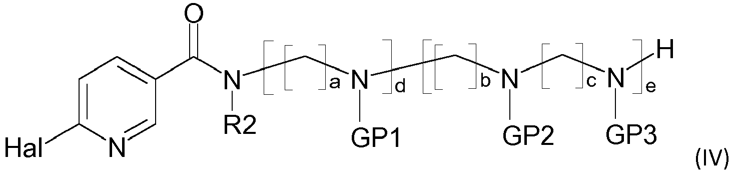 wherein R2, a, b, c, d and e are as defined in claim 1, GP1, GP2 and GP3 are as defined above and Hal represents a halogen atom,
(b) reacting the compound having formula (IV) obtained in step (a) above with hydrazine to obtain a compound having the following formula (V): 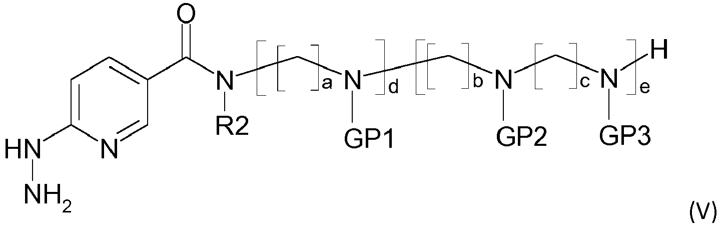 wherein R2, a, b, c, d and e are as defined in claim 1 and GP1, GP2 and GP3 are as defined above,
(c) optionally protecting, with an N-protecting group, the hydrazine function of the compound having formula (V) obtained in step (b) above to obtain a compound having the following formula (VI): 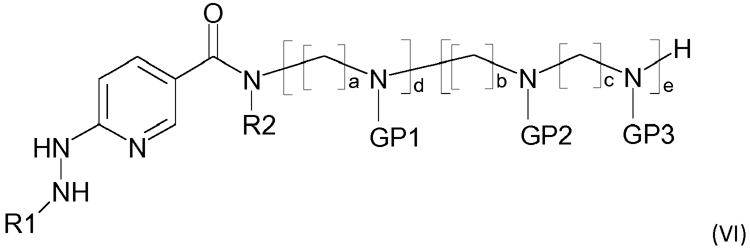 wherein R2, a, b, c, d and e are as defined in claim 1, GP1, GP2 and GP3 are as defined above and R1 represents an N-protecting group as defined in claim 1,
(d) deprotecting the amine functions protected by the groups GP1, GP2 and GP3 in the compound having formula (V) or (VI) obtained in step (b) or (c) above to obtain a compound having formula (I),
(e) optionally salifying the compound having formula (I) obtained in step (d) above to obtain a pharmaceutically acceptable salt thereof, and
(f) separating the compound having formula (I) or the pharmaceutically acceptable salt thereof obtained in the previous step from the reaction medium.

15. A process for preparing a compound having formula (I) according to any one of claims 1 to 6 wherein R1 ≠ H or a pharmaceutically acceptable salt thereof comprising the following successive steps:
(i) protecting, with an N-protecting group, the hydrazine function of 6-hydrazinyl-nicotinic acid to obtain a compound having the following formula (VII): 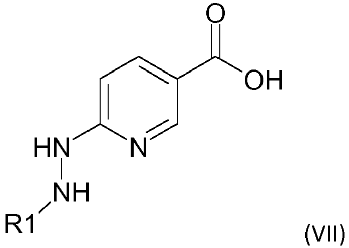 wherein R1 represents an N-protecting group as defined in claim 1,
(ii) reacting the compound having formula (VII) obtained in step (i) above with a protected polyamine having formula (III) as defined in claim 14 to obtain a compound having formula (VI) as defined in claim 14,
(iii) deprotecting the amine functions protected by the groups GP1, GP2 and GP3 in the compound having formula (VI) obtained in step (ii) above to obtain a compound having formula (I) wherein R1 ≠ H,
(iv) optionally salifying the compound having formula (I) obtained in step (iii) above to obtain a pharmaceutically acceptable salt thereof, and
(v) separating the compound having formula (I) or the pharmaceutically acceptable salt thereof obtained in the previous step from the reaction medium.

16. A process for preparing a complex having formula (II) according to any one of claims 7 and 8 or a pharmaceutically acceptable salt thereof comprising the mixture of a compound having formula (I) according to any one of claims 1 to 6 or a pharmaceutically acceptable salt thereof with a pertechnetate-99m salt, at least one reducing agent and tricine.
